(19) Europäisches Patentamt
European Patent Office
Office européen des brevets

(11) EP 3 273 794 B1

(12) EUROPEAN PATENT SPECIFICATION

(45) Date of publication and mention of the grant of the patent:
**07.08.2019 Bulletin 2019/32**

(21) Application number: **16710496.7**

(22) Date of filing: **17.03.2016**

(51) Int Cl.:
***A23K 10/20*** *(2016.01)* ***A23K 50/42*** *(2016.01)*
***A23K 40/25*** *(2016.01)*

(86) International application number:
**PCT/IB2016/051516**

(87) International publication number:
**WO 2016/151440 (29.09.2016 Gazette 2016/39)**

# (54) METHOD USING FRESH OR FROZEN MEAT TO IMPROVE FOOD EFFICIENCY IN ANIMALS SUCH AS DOGS

VERFAHREN ZUR VERWENDUNG VON FRISCH- ODER TIEFKÜHLFLEISCH ZUR VERBESSERUNG DER NAHRUNGSEFFIZIENZ BEI TIEREN WIE ETWA HUNDEN

PROCÉDÉ UTILISANT DE LA VIANDE FRAÎCHE OU CONGELÉE POUR AMÉLIORER L'EFFICACITÉ ALIMENTAIRE D'ANIMAUX TELS QUE DES CHIENS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2015 US 201562136701 P**

(43) Date of publication of application:
**31.01.2018 Bulletin 2018/05**

(73) Proprietor: **Société des Produits Nestlé S.A.**
**1800 Vevey (CH)**

(72) Inventors:
- **BOUTHEGOURD, Jean-Christophe**
  **F95110 Sannois (FR)**
- **ROOS, Mark Alan**
  **Columbia, Illinois 62236 (US)**
- **CLETY, Nathalie**
  **80440 Boves (FR)**
- **DOARE-BROUX, Karine**
  **75010 Paris (FR)**
- **LORCY, Gwendal**
  **80800 Amiens (FR)**

(74) Representative: **Elkington and Fife LLP**
**Prospect House**
**8 Pembroke Road**
**Sevenoaks, Kent TN13 1XR (GB)**

(56) References cited:
**WO-A1-2006/062519 US-A1- 2009 304 897**
**US-A1- 2012 237 642 US-A1- 2013 029 027**

- **DATABASE WPI Week 201512 Thomson Scientific, London, GB; AN 2015-11075X XP002757652, & CN 104 206 655 A (XUZHOU FURUN BIRDS FOOD CO LTD) 17 December 2014 (2014-12-17)**
- **GRECO ET AL: "Nutritional supplements for pregnant and lactating bitches", THERIOGENOLOGY, LOS ALTOS, CA, US, vol. 70, no. 3, 1 August 2008 (2008-08-01) , pages 393-396, XP022776667, ISSN: 0093-691X, DOI: 10.1016/J.THERIOGENOLOGY.2008.04.013 [retrieved on 2008-06-12]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 273 794 B1

## Description

BACKGROUND

The present disclosure relates generally to dry pet food products and methods for making dry pet food products. More specifically, the present disclosure relates to methods and compositions that improve availability of amino acids and fatty acids.

Dietary protein is required to provide essential amino acids and nitrogen for the synthesis of non-essential amino acids, endogenous proteins, other nitrogenous compounds, and also to provide energy. An adapted level of high quality protein is essential for canine health: strengthening muscles and helping to maintain lean body mass, for healthy skin and coat, to support the immune system, and all vital organ function.

During gestation and lactation, a female dog's nutrient and energy requirements are greatly increased. In fact, during lactation, energy requirements are higher than at any other time in the female dog's life. Lactation is a time when weight loss is usual and when female dogs can lose several points of body condition score. To meet the increased nutrient and energy requirements, a high quality diet is advised.

Animal meats and animal by-products such as raw lamb, ground beef, or chicken meal are sources of proteins commonly used by pet food manufacturers because of their high digestibility and palatability. In the pet food industry, dry or dried ingredients are normally used for production of dry pet foods by extrusion processing. Raw animal meat, bone, and by-products are rendered by a blending, heating and drying process in order to make a dry protein-rich meal.

Compared to these rendered meals, addition of raw animal by-products to a pet food recipe adds complexity and costs in terms of processability. For example, poultry by-products meal is provided as a dried material by a supplier and can be directly incorporated into a dry mix that is extruded to form the pet food. In contrast, cooking raw chicken carcass is time- and space-consuming for pet food manufacturers. Therefore, the production of a pet food with a raw animal protein source is more expensive than without such an ingredient, and thus commercial pet food is typically based on rendered meals as the protein source. US2012/0237642 A1 relates to a method for production of a meat-based, semi-moist animal food product by extrusion, comprising the steps of: charging an extruder with at least one component of the group dry meat, meat meal, dry fish, fish meal, and meat and/or fish.

SUMMARY

The present disclosure relates to methods and compositions for improving amino and fatty acids availability that result in better blood enrichment in limiting amino acids, such as arginine and lysine, and essential fatty acids, such as linoleic acid. The present inventors unexpectedly observed a higher fat digestibility when rendered meat meal in pet food was replaced with a fresh or frozen meat slurry. To the best knowledge of the inventors, this effect has not been documented in previous publications. Better growth rates of puppies and better weight maintenance of adults were observed when rendered meat meal in the food was replaced with up to 20% fresh or frozen meat slurry.

As detailed in the experimental evidence set forth herein, the present inventors were able to measure the following health benefits during feeding trials: (i) a trend for a better weight maintenance during very cold temperature exposure in dogs fed with a product containing 20% meat slurry or a product containing 14% meat slurry, relative to dogs fed with a product having the same total protein but not containing a meat slurry; (ii) better weight maintenance in gestating and lactating female dogs fed with a puppy diet containing 14% meat slurry vs. a puppy diet having the same total protein but containing no meat slurry; (iii) better growth rate in lactating puppies of the female dogs fed the 14% meat slurry diet vs. the diet having the same total protein but without meat slurry therein; (iv) higher milk protein content in female dogs fed the 14% meat slurry diet vs. the diet having the same total protein but without meat slurry therein; (v) higher milk enrichment in essential and non-essential amino acids in female dogs fed the 14% meat slurry therein; and (vi) higher diet efficiency in promoting a high quality milk as demonstrated by higher levels of essential amino acids vs. diet essential amino acids content in the 14% meat slurry diet. Without being bound by theory, the present inventors believe these effects are due to a better availability of essential amino acids and essential fatty acids in diets formulated with a meat slurry.

Accordingly, in a general embodiment, a method of producing a dry pet food composition is provided. The method comprises: forming a meat slurry from fresh or frozen meat; adding the meat slurry to a dry blend of ingredients to form a mixture in which the meat slurry is about 14% to about 20% of the mixture; subjecting the mixture to extrusion cooking to form an extrudate; and processing the extrudate to form the pet food composition, wherein processing the extrudate comprises cutting the extrudate into pieces and drying the pieces.

In an embodiment, the forming of the meat slurry comprises subjecting the fresh or frozen meat to size reduction and cooking the size-reduced fresh or frozen meat with direct steam injection at a temperature of about 71 °C or less. The forming of the meat slurry can comprise emulsifying the steam-injected meat.

In an embodiment, the mixture comprises about 18% to about 30% protein.

In an embodiment, the dry blend comprises a whole grain.

In an embodiment, the dry blend comprises a fiber.

In an embodiment, the meat slurry is added to the dry blend of ingredients in a preconditioner, and the mixture is fed from the preconditioner to an extruder that performs the extrusion cooking. The extrusion cooking can be performed in the extruder at a temperature of 105 to 130 °C at a pressure of 1724 to 3447 kPa (250 to 500 psi) for a time period less than 40 seconds.

In an embodiment, the processing of the extrudate comprises cutting the extrudate into pieces and drying the pieces. The drying of the pieces can reduce a moisture content of the pieces to about 6% to about 9% moisture. The processing of the extrudate comprises coating the dried pieces with at least one of an animal fat or an animal digest.

In another embodiment, the present disclosure provides a dry pet food composition. The composition comprises 14% to 20% of meat that is not meat meal, wherein the meat that is not meat meal is a meat slurry from fresh or frozen meat.

In an embodiment, the dry pet food composition has a protein content of about 18% to about 30%. The protein content can be fully provided by the meat slurry and a protein source selected from the group consisting of a vegetable protein, a meat meal, and combinations thereof.

In an embodiment, the meat that is not meat meal is provided by making the dry pet food composition with a process comprising subjecting fresh or frozen meat to size reduction, cooking the size-reduced fresh or frozen meat with direct steam injection, emulsifying the steam-injected meat to form a slurry, and adding the slurry to one or more other ingredients.

In another embodiment, the present disclosure provides a method of improving bioavailability of at least one of an essential fatty acid or an essential amino acid relative to an initial dry pet food formulation comprising a meat meal that provides at least a portion of a protein content of the initial dry pet food formulation. The method comprises: adjusting the initial dry pet food formulation to replace at least a portion of the meat meal with meat provided by meat that is not meat meal, wherein the meat that is not meat meal is a meat slurry from fresh or frozen meat, wherein the meat slurry is 14 wt% to 20 wt% of the adjusted dry pet food formulation, wherein the adjusted dry pet food formulation has a protein content that is about equal to the protein content of the initial dry pet food formulation; and producing a kibble according to the adjusted dry pet food formulation.

In an embodiment, the initial dry pet food formulation comprises non-meat ingredients, and the adjusted dry pet food formulation has the same amount of the non-meat ingredients relative to the initial dry pet food formulation.

An advantage of the present disclosure is to provide pet food formulated with an ingredient that fully sustains availability of amino acids for protein synthesis

An advantage of the present disclosure is to provide pet food formulated with an ingredient that brings high quality protein and high level of essential fatty acids.

Yet another advantage of the present disclosure is to provide pet food formulated with an ingredient that is proven to be highly digestible.

Another advantage of the present disclosure is to provide pet food formulated with an ingredient that helps prevent weight loss in a challenging environment.

Another advantage of the present disclosure is to provide pet food formulated with an ingredient that is proven to deliver superior nutrition for long term health impact.

Another advantage of the present disclosure is to provide pet food formulated with an ingredient that helps maintain body condition in dogs living in kennels.

Another advantage of the present disclosure is to provide pet food formulated with an ingredient that helps maintain body weight in healthy adult dogs.

Another advantage of the present disclosure is to provide pet food formulated with an ingredient that provides key nutritional benefits being a high quality protein and fat source.

An advantage of the present disclosure is to provide pet food formulated with an ingredient that is an improved source of essential amino acids and essential fatty acids to grow strong puppies.

Another advantage of the present disclosure is to provide a pet food product that helps grow a strong and healthy litter.

Yet another advantage of the present disclosure is to provide a high quality animal protein source for a strong and healthy growth.

Still another advantage of the present disclosure is to improve availability of essential nutrients in pet food.

Another advantage of the present disclosure is to help maintain a healthy body weight in a gestating/lactating female dog.

Yet another advantage of the present disclosure is to provide a pet food that contains all the nutrients needed by a gestating/lactating female dog to grow strong puppies.

Still another advantage of the present disclosure is to provide a pet food that contains all the nutrients needed by a puppy to become a strong adult.

Yet another advantage of the present disclosure is to provide a pet food that achieves a high availability of essential amino acids in a lactating female dog's milk.

Additional features and advantages are described herein, and will be apparent from, the following Detailed Description and the Figures.

## BRIEF DESCRIPTION OF THE FIGURES

**FIG. 1A** is a graph showing the plasmatic concentration of lysine over time from the study in Example 1.

**FIG. 1B** is a graph showing the plasmatic concentration of arginine over time from the study in Example 1.

**FIG. 2** is a graph showing the fatty acid profile from the three diets investigated in the study in Example 2.

**FIG. 3** is a graph showing the amino acid profile from the three diets investigated in the study in Example 2.

**FIG. 4** is a graph showing the digestibilities of the three diets investigated in the study in Example 2.

**FIG. 5** is a graph showing the apparent absorbed linoleic acid from the three diets investigated in the study in Example 2.

**FIG. 6** is a graph showing the lysine enrichment from the three diets investigated in the study in Example 2.

**FIG. 7** is a graph showing the arginine enrichment from the three diets investigated in the study in Example 2.

**FIG. 8** is a graph showing the body weight lost during extreme weather conditions by dogs fed with the three diets investigated in the study in Example 2.

**FIG. 9** is a graph showing the product digestibility of the two diets investigated in the study in Example 3.

**FIGS. 10A** and **10B** are graphs showing the body weight evolution of mothers during the late gestation and lactation periods with the two diets investigated in the study in Example 3.

**FIG. 11** is photographs showing the body weight evolution during the late gestation and lactation periods of a border collie mother fed with Diet A investigated in the study in Example 3.

**FIGS. 12A** and **12B** are graphs showing the puppies' growth during lactation with the two diets investigated in the study in Example 3.

**FIG. 13** is a graph showing the puppies' food efficiency during lactation with the two diets investigated in the study in Example 3.

**FIGS. 14A** and **14B** are graphs showing the French Bulldog puppies' growth during lactation with the two diets investigated in the study in Example 3.

**FIGS. 15A-15C** and **16** are graphs showing the milk quality, as rated by breeders, of the lactating female dogs fed with the two diets investigated in the study in Example 3.

**FIG. 17** is a graph showing the milk protein content of the lactating female dogs fed with the two diets investigated in the study in Example 3.

**FIG. 18** is a graph showing the milk protein content of the lactating female Bulldogs fed with the two diets investigated in the study in Example 3, along with published data regarding Beagles.

**FIG. 19** is a graph showing the amino acid profile in milk from dogs fed Diets A and B.

**FIGS. 20A, 20B** and **20C** are graphs showing the correlation between amino acid enrichment in milk vs. dietary amino acid level in dogs fed Diets A and B at Early, Mid and Late lactation periods.

## DETAILED DESCRIPTION

As used in this disclosure and the appended claims, the singular forms "a," "an" and "the" include plural referents unless the context clearly dictates otherwise. Thus, for example, reference to "a slurry" or "the slurry" includes two or more slurries. The term "and/or" used in the context of "X and/or Y" should be interpreted as "X," or "Y," or "X and Y." Similarly, the recitation "at least one of X or Y" should be interpreted as "X," or "Y," or "X and Y." Where used herein, the term "example," particularly when followed by a listing of terms, is merely exemplary and illustrative, and should not be deemed to be exclusive or comprehensive.

As used herein, "about" is understood to refer to numbers in a range of numerals, for example the range of -10% to +10% of the referenced number, preferably within -5% to +5% of the referenced number, more preferably within -1% to +1% of the referenced number, most preferably within -0.1% to +0.1% of the referenced number. Furthermore, all numerical ranges herein should be understood to include all integers, whole or fractions, within the range. Moreover, these numerical ranges should be construed as providing support for a claim directed to any number or subset of numbers in that range. For example, a disclosure of a range from 1 to 10 should be construed as supporting a range of from 1 to 8, from 3 to 7, from 1 to 9, from 3.6 to 4.6, from 3.5 to 9.9, and so forth.

All percentages expressed herein are by weight of the total weight of the composition unless expressed otherwise. When reference is made to the pH, values correspond to pH measured at 25 °C with standard equipment.

The terms "food," "food product" and "food composition" mean a product or composition that is intended for ingestion by an animal, including a human, and provides at least one nutrient to the animal. The term "pet food" means any food composition intended to be consumed by a pet. The term "pet" means any animal which could benefit from or enjoy the compositions provided by the present disclosure. For example, the pet can be an avian, bovine, canine, equine, feline, hicrine, lupine, murine, ovine, or porcine animal, but the pet can be any suitable animal. The term "companion animal" means a dog or a cat. Maturation of juvenile dogs occurs at different rates for different species, but the term "puppy" as used herein means a dog in the first two years following birth, preferably the 18 months following birth, more preferably the one year following birth.

A "dry" food composition has less than 10% moisture and/or a water activity less than 0.65, preferably both. "Kibbles" are pieces of dry pet food which can have a pellet shape or any other shape. Examples of kibbles include particulates; pellets; pieces of petfood, dehydrated meat, meat analog, vegetables, and combinations thereof; and pet snacks, such as meat or vegetable jerky, rawhide, and biscuits. The present disclosure is not limited to a specific form of the kibbles.

As used herein, "meat meal" is meat that has been dried and ground to form substantially uniform-sized particles. For example, the Association of American Feed Control Officials (AAFCO) defines "meat meal" as the rendered product from mammal tissues, exclusive of any added blood, hair, hoof, horn, hide trimmings, manure, stomach and rumen contents except in such amounts as may occur unavoidably in good processing practices, and shall not contain extraneous materials not provided for by this definition.

The compositions disclosed herein may lack any element that is not specifically disclosed herein. Thus, a disclosure of an embodiment using the term "comprising" includes a disclosure of embodiments "consisting essentially of' and "consisting of' the components identified. Similarly, the methods disclosed herein may lack any step that is not specifically disclosed herein. Thus, a disclosure of an embodiment using the term "comprising" includes a disclosure of embodiments "consisting essentially of' and "consisting of' the steps identified. Any embodiment disclosed herein can be combined with any other embodiment disclosed herein.

In an aspect of the present disclosure, a food composition comprises up to about 20% meat that is not meat meal. Preferably, the food composition is a dry food composition. The food composition can be a pet food, for example a food composition formulated for dogs. In an embodiment, the amount of the meat that is not meat meal is from about 14% to about 20% of the food composition, for example about 16%. The food composition preferably has a protein content of about 18% to about 30%, preferably about 25% to about 30%, for example about 25%. In an embodiment, the food composition has a fat content of about 10% to about 20%, preferably about 15% to about 17%. The food composition preferably has an ash content of about 6% to about 8%.

The food composition preferably is made by a process that comprises producing a slurry of fresh or frozen meat. For example, fresh or frozen meat can be subjected to size reduction, cooking with direct steam injection to increase the moisture of the meat, and emulsification to form the slurry. Examples of suitable meats include chicken, beef, pork, lamb, turkey, rabbit, duck, goose, and fish such as salmon, tuna, mackerel, cod, pollock, halibut, sole and haddock. The fresh or frozen meat can be a single type of meat or a combination of two or more types of meat.

The direct steam injection preferably increases the moisture of the meat by adding 5 to 15 kg of hot water/steam per 100 kg of meat. In an embodiment, the temperature of the slurry is about 71 °C, although in some embodiments lower temperatures can be used. Preferably the resultant slurry has a viscosity that does not prevent the slurry from being pumped through injectors into a pre-conditioner that hydrates and mixes the materials before extrusion.

A specific example of slurry preparation is provided hereafter. Frozen meat blocks can be sent to a pre-breaker in order to start size reduction of frozen blocks to chunks generally from 5 cm to 15 cm in size. These meat chunks can be conveyed to a grinder for size reduction to pieces of 6 mm or 8 mm on their longest side. If fresh meat is used additionally or alternatively to the frozen meat, the fresh meat can be added to the grinder directly. The ground meat can be cooked by direct steam injection to a temperature of about 71°C for 5 - 15 seconds to form a slurry, preferably under constant agitation through heating and any subsequent holding. The slurry can undergo a final size reduction and an emulsification while the slurry temperature is maintained at about 71 °C or less using a standard emulsifier. The final size reduction and the emulsification can allow pumping and injecting into a pre-conditioner prior to extrusion-cooking.

To produce the food composition, the slurry can be added to a blend of dry ingredients. In an embodiment, the slurry is added to at least a portion of the blend of dry ingredients in the preconditioner, and the mixture is fed from the preconditioner to the extruder.

The amount of the slurry can be determined based on the desired amount in the final food composition, preferably about 14% to about 20% in the food composition. The concentrations of the ingredients in the mixture formed by adding the slurry to the dry blend can be substantially the same as the concentrations in the final composition (other than water content), although typically the concentrations will be slightly less in the final composition due to moisture addition in the preconditioner, a portion of which remains in the final composition. Moreover, the amount of the slurry added into a product formula is preferably 105% - 115% of the desired amount of meat from the slurry, due to the hot water/steam added during cooking the meat. Hence, for a formula requiring 14% chicken when 10 kg water is added to 100 kg chicken to bring the temperature up to about 71 °C, an amount of about 15.4% of the chicken slurry should be added.

The dry blend can contain one or more of meat meals, grains, vegetable proteins, fiber, vitamins, minerals and fats. Examples of meat meals suitable for the compositions disclosed herein include beef meal, poultry meal, pork meal, turkey meal, fish meal and combinations thereof. To maintain the desired level of total protein in the product, an increase in the slurry level is accompanied by a decrease in the amount of any animal meal present. In an embodiment, the amount of the slurry is greater than the amount of any meat meal, although other embodiments have an amount of meat meal that is greater than the amount of the slurry.

Examples of suitable grains include corn, rice, wheat, barley, oats, soy, sorghum, millet, triticale, rye and mixtures thereof, preferably in whole grain form. In an embodiment, an amount of grain is used so that the final food composition comprises 20-55% of the grain.

Examples of suitable vegetable proteins include wheat protein (e.g., whole grain wheat or wheat gluten such as vital wheat gluten), corn protein (e.g., ground corn or corn gluten), soy protein (e.g., soybean meal, soy concentrate, or soy isolate), rice protein (e.g., ground rice or rice gluten), cottonseed, peanut meal, pea protein, and combinations thereof. Some materials are both a vegetable protein and a grain. In an embodiment, an amount of vegetable protein is used so that the final food composition comprises 5-20% of the vegetable protein.

Soluble fibers and/or insoluble fibers may be utilized. Examples of suitable fiber sources include chicory, cellulose, beet pulp (from sugar beet), gum arabic, gum talha, psyllium, rice bran, carob bean gum, citrus pulp, pectin, fructooligosaccharide, short chain oligofructose, mannanoligofructose, soy fiber, arabinogalactan, galactooligosaccharide, arabinoxylan, and mixtures thereof. In an embodiment, an amount of fiber is used so that the final food composition comprises 1-10% of the fiber.

The fiber source can be a fermentable fiber. Fermentable fiber has previously been described to provide a benefit to the immune system of a companion animal. Fermentable fiber or other compositions known to skilled artisans that provide a prebiotic to enhance the growth of probiotics within the intestine may be incorporated into the dry pet food.

Examples of suitable fats include animal fats and vegetable fats. Preferably the fat source is an animal fat source, such as tallow or grease. Vegetable oils, such as corn oil, sunflower oil, safflower oil, rape seed oil, soy bean oil, olive oil and other oils rich in monounsaturated and polyunsaturated fatty acids, can be used additionally or alternatively. In some embodiments, a source of omega-3 fatty acids is included, such as one or more of fish oil, krill oil, flaxseed oil, walnut oil, or algal oil.

Examples of suitable vitamins include vitamin A, any of the B vitamins, vitamin C, vitamin D, vitamin E, and vitamin K, including various salts, esters, or other derivatives of the foregoing. Non-limiting examples of suitable minerals include calcium, phosphorous, potassium, sodium, iron, chloride, boron, copper, zinc, magnesium, manganese, iodine, selenium, and the like.

The dry blend can include other additional components such as one or more of a preservative, a colorant or a palatant. Examples of suitable preservatives include potassium sorbate, sorbic acid, sodium methyl para-hydroxybenzoate, calcium propionate, propionic acid, and combinations thereof. Examples of suitable colorants include FD&C colors, such as blue no. 1, blue no. 2, green no. 3, red no. 3, red no. 40, yellow no. 5, yellow no. 6, and the like; natural colors, such as roasted malt flour, caramel coloring, annatto, chlorophyllin, cochineal, betanin, turmeric, saffron, paprika, lycopene, elderberry juice, pandan, butterfly pea and the like; titanium dioxide; and any suitable food colorant known to the skilled artisan. Examples of suitable palatants include yeast, tallow, rendered animal meals (e.g., poultry, beef, lamb, and pork), flavor extracts or blends (e.g., grilled beef), animal digests, and the like.

An example of a method of using the slurry to produce a food composition follows hereafter. A dry pet food using a slurry of a meat that is one or more of chicken, turkey, beef, lamb or salmon can be made by adding about 14% to about 20% of the meat slurry, for example about 16% of the meat slurry, to a ground dry blend. The ground dry blend can comprise about 10% to about 30% of poultry meal and/or meal from another animal, for example about 20% of the animal meal; about 20% to about 55% of a whole grain mixture comprising corn, wheat and milled rice, for example about 46% of the whole grain mixture; about 5% to about 35% of a vegetable protein mixture comprising soy, corn gluten, and wheat gluten, for example about 10% of the vegetable protein mixture; about 1% to about 15% of a fiber mixture comprising chicory, cellulose and beet pulp, for example about 6% of the fiber mixture; about 0.5% to about 2.0% of vitamins and minerals, for example about 1.0% of the vitamins and minerals; and about 0.1% to about 3.0% of fish oil, for example about 1.0% of the fish oil.

The meat slurry can be added to the dry blend in a preconditioner with continuous agitation and brought up to a temperature of 80 - 85 °C with steam injection. The preconditioned mix can be fed to an extruder-cooker and processed at a temperature of 105 - 130 °C and a pressure of 1724 to 3447 kPa (250 - 500 psi) for a time period less than 40 seconds to form shaped expanded ropes. The ropes can be cut to suitably sized pieces. The pieces can be dried to 6 - 9% moisture to form kibbles, and the kibbles can be coated with about 8% of animal fat and digest.

Also described herein is a method of providing nutrition to a pet is provided. The pet can be a companion animal, preferably a dog, more preferably a pregnant and/or lactating female dog. The method comprises administering any of the embodiments of the pet food product disclosed herein. In a preferred embodiment, the pet food product is administered to a pregnant and/or lactating dog daily during the pregnancy and/or daily during the lactation period to improve the growth of the puppy and to support weight maintenance of the mother.

**EXAMPLES**

The following examples are illustrative of the concept of using fresh or frozen meat to improve availability of amino acids and fatty acids.

EXAMPLE 1

A wide investigation of the protein quality of the ingredients chicken carcass (CC) and poultry by-products meal (PBPM) was made. CC and PBPM were (i) analyzed for chemical composition; (ii) analyzed for *in vitro* protein digestibility; and (iii) included in dog diets and evaluated for postprandial amino acid (AA) plasmatic appearance and apparent total tract digestibility. Based on the latter outcomes, the ileal AA digestibility was estimated and related to the postprandial AA plasmatic appearance. Experimental ingredients came from the same batch for all experiments.

Chemical Characterization

CC and PBPM were analyzed for moisture, crude protein (CP), crude fat, crude fiber, ash, and gross energy (GE). Measurements of methionine sulfone, methionine sulfoxide, and cysteic acid were conducted on the experimental diets and ingredients by ion-exchange chromatography and colorimetric detection after post-column derivatisation with ninhydrin reagent following the European Union regulation. In addition, the non-reactive lysine of CC, PBPM, CC diet, and PBPM diet was estimated.

Collagen in the experimental ingredients and experimental diets was analyzed using a method with sulphuric acid and potassium hydroxide. The CC diet and the PBPM diet for dogs were analyzed for moisture, CP, crude fat, crude fiber, ash, and gross energy (GE). Essential Amino Acids (EAA) were analyzed as well. Moreover, CC and PBPM were evaluated for *in vitro* protein digestibility using the Lareal/Boisen method.

Table 1: Ingredient composition of the experimental dog diets

| Animal Protein source | CC Diet | PBPM Diet |
|---|---|---|
| Ingredients (%) | | |
| Chicken Carcass | 28.13 | - |
| Poultry by-products | - | 6.42 |
| Fat pork premier jus | 2.00 | 5.00 |
| Corn grain common | 72.03 | 73.50 |
| Gluten corn meal | 11.60 | 11.50 |
| Chicken digest | 2.00 | 2.00 |
| Minerals | 5.10 | 5.71 |
| Vitamins | 0.34 | 0.35 |
| Antioxidants | 0.0016 | 0.0040 |

Table 2: Chemical composition of the experimental dog diets

| Animal Protein source | CC Diet | PBPM Diet |
|---|---|---|
| Dry Matter | 91.9 | 91.6 |
| Ingredients (% of dry matter) | | |
| Crude protein | 18.87 | 18.03 |
| Fat | 11.2 | 9.2 |
| Carbohydrate (NFE) | 53.23 | 55.67 |
| Crude fiber | 1.7 | 2.1 |

(continued)

| Ingredients (% of dry matter) | | |
|---|---|---|
| Ash | 6.9 | 6.6 |
| Collagen (% of CP) | 12.2 | 10.0 |
| ME, Kcal/g | 3.45 | 3.35 |
| ME, KJ/g | 14.44 | 14.00 |
| Protein (% of ME) | 17.6 | 17.3 |
| Fat (% of ME) | 25.3 | 21.4 |
| Essential Amino Acids | | |
| Arginine | 0.87 | 0.87 |
| Cysteine | 0.31 | 0.32 |
| Histidine | 0.43 | 0.43 |
| Isoleucine | 0.68 | 0.72 |
| Leucine | 2.28 | 2.38 |
| Lysine | 0.64 | 0.63 |
| Methionine | 0.33 | 0.32 |
| Phenylalanine | 0.98 | 1.02 |
| Threonine | 0.66 | 0.66 |
| Tryptophan | 0.12 | 0.12 |
| Valine | 0.82 | 0.86 |
| Non-Essential Amino Acids | | |
| Alanine | 1.47 | 1.48 |
| Aspartic Acid | 1.25 | 1.26 |
| Glutamic Acid | 3.41 | 3.51 |
| Glycine | 1.10 | 1.00 |
| Proline | 1.58 | 1.56 |
| Serine | 0.92 | 0.93 |
| Total Essential AA (TEAA) | 8.12 | 8.33 |
| Total Non-Essential AA (TNEAA) | 9.73 | 9.74 |
| Total AA (TAA) | 17.85 | 18.07 |

Table 3: Chemical composition of chicken carcass and poultry by-products meal

| Animal Protein Source | CC | PBPM |
|---|---|---|
| Dry Matter | 39.4 | 94.0 |
| Ingredients (% of dry matter) | | |
| Crude protein | 38.68 | 69.38 |
| Fat | 47.21 | 14.38 |
| Crude fiber | 2.79 | 1.5 |
| Ash | 14.97 | 14.3 |

(continued)

| Ingredients (% of dry matter) | | |
|---|---|---|
| Collagen (% of CP) | 39.4 | 29.7 |
| ME, Kcal/g | 49.30 | 84.03 |
| ME, KJ/g | 206.4 | 351.80 |
| Essential Amino Acids | | |
| Arginine | 1.01 | 4.44 |
| Cysteine | 0.13 | 0.64 |
| Histidine | 0.32 | 1.25 |
| Isoleucine | 0.48 | 2.41 |
| Leucine | 0.95 | 4.35 |
| Lysine | 0.93 | 3.96 |
| Methionine | 0.24 | 1.23 |
| Phenylalanine | 0.53 | 2.47 |
| Threonine | 0.53 | 2.52 |
| Tryptophan | 0.11 | 0.61 |
| Valine | 0.62 | 2.83 |
| Non-Essential Amino Acids | | |
| Alanine | 1.08 | 4.29 |
| Aspartic Acid | 1.15 | 5.16 |
| Glutamic Acid | 1.80 | 7.67 |
| Glycine | 1.72 | 5.97 |
| Proline | 1.13 | 4.32 |
| Serine | 0.57 | 2.75 |
| Total Essential AA (TEAA) | 6.19 | 28.60 |
| Total Non-Essential AA (TNEAA) | 7.45 | 30.16 |
| Total AA (TAA) | 13.64 | 58.76 |

Dog Assay

Sixteen dogs were used for the study with various breeds, gender and sterilized status: 7 Beagles, 2 Cairns Terriers, 2 Dachshunds, 4 Fox Terriers, and 1 Mini Schnauzer. Dogs (aged 4.92 ± 2.92 years; 9.0 ± 2.3 kg body weight) were individually housed in indoor kennels with continual free access to a large external court. Room temperature in the facility was maintained between 18 and 24 °C with a 12-h light/ 12-h dark cycle. All dogs were fed the diets to maintain body weight throughout the experiment. Diets were given in one single portion at 09.00. Dogs had continuous access to water.

The diets were formulated to meet or exceed the minimum requirements for adult dogs established by The European Pet Food Industry Federation (FEDIAF). The ingredient composition of the two diets is reported above in Table 1. Values are presented on a dry matter (DM) basis. Diets differed in the composition by the sole source of animal protein. CC and PBPM were respectively the sole source of animal protein of the CC diet and the PBPM diet. CC and PBPM contribution in CP was formulated to be identical, about 25%, in both diets. Approximately 73% of the protein was provided by corn grain and gluten corn meal. Diets were formulated to contain a similar proportion, on a DM basis, of CP, fat, crude fiber, carbohydrate (nitrogen-free extract, NFE), and ash (Table 2). CC was stored frozen before grinding and cooking for 12min at 70/80°C. The product resulting from that process, called the slurry, was then incorporated into the extruder with the other ingredients. Diets were extruded through an extruder, dried for about 20/25 min at 110°C to a target of 8% DM, and then kibbled.

Dog health and behavior was monitored every day by the colony veterinarians, an animal behaviorist and/or the caretaker staff. If needed, medical treatments were administered. All dogs were considered healthy based on results of physical examination and clinical laboratory tests.

The study was conducted for 26 days in a crossover design. During the first period, the first group of 8 dogs was fed the CC diet while the other group of 8 dogs was fed the PBPM diet. During the second period, the first group of dogs was allowed the PBPM diet when the second group the CC diet. Per period, dogs were allowed a 7-day adaptation period after which a 6-day total fecal collection was conducted for determination of nutrient digestibilities. Food consumption was measured daily. When collected, wet feces were stored at -20°C until the completion of the 7-d collection period and weighed at the end that period. Diet and fecal samples were analyzed for moisture, CP, crude fat, crude fiber, ash, and gross energy (GE). Total tract nutrient digestibilities were calculated as: [nutrient intake (total g/7 d) - nutrient output (total g/7 d)]/nutrient intake (total g/7 d).

Dogs from the digestibility test were used during a day for blood sampling during the pre- and postprandial periods. 12 dogs among the 16 dogs per diet were chosen for their ability to eat in a short period of time. The AA absorption test occurred once per dog during the fecal collection of each period. Blood samples (3 mL) were collected through the cephalic catheter into two 2.5-mL syringes using a 1" x 20 g needle. Immediately prior to collecting the 3-mL blood samples, 0.5 mL of blood was collected and discarded to remove residual anticoagulant in the catheter. Blood was collected 0.5 h before feeding (time 0), and at 1, 2, 3, 5, and 8 h after feeding time. Following collection, blood was immediately transferred into a 5-mL Na-heparin tube. A 1.0 mL volume of heparinized saline (10 U heparin/mL saline) was given after each collection to flush the catheter. After the 8 h sample the catheter was removed and the 24 h sample was collected by venipuncture of the jugular vein. In total, per period, 24.5 mL of blood was collected, which is below the GIRCOR (Interprofessional Group on Reflection and Communication on Research) recommendations. Blood was centrifuged at 4,000 x g for 5 min at room temperature in a clinical centrifuge. The supernatant fraction obtained was removed and then stored at -80°C until amino acids analysis. Plasma AA concentrations were performed with Biochrom amino acid analysers using classical ion-exchange liquid chromatography with post-column Ninhydrin derivatisation and photometric detection. Area under the curve (AUC) was calculated for 24 hours using trapezoidal rule: $\mathrm{AUC} = \int_a^b f(x)dx.$

Variables were tested for normality and appeared to adhere to a normal distribution. A paired *t*-test was used to test the significance of the apparent total tract digestibilities. Differences were considered significantly different when the test had a p-value less than 0.05. Statistical tests were all two-tailed. Areas under the curve were analyzed by analysis of variance. The results are presented as mean ± SD.

Results

CC and PBPM were analyzed for *in vitro* protein digestibility and collagen content. Assays on dogs were conducted on produced diets to obtain information in the relative quality of the CC and the PBPM. Finally, diets were fed to dogs in order to obtain apparent total tract nutrient digestibility including single amino acid digestibility data. These ileal estimated AA digestibilities were compared to the postprandial plasmatic enrichment in EAAs in order to have a qualitative comparison between both diets in terms of AA absorption. These *in vivo* data together with analytical characterization of both ingredients and diets should permit to conclude on the quality of the protein sources and give a good estimation of their EAA availability in dogs.

Chemical Characterization: Chemical compositions of the experimental ingredients are shown above in Table 3. Non-reactive lysine proportions were similar among the experimental ingredients and experimental diets with values varying between 14.8 and 18% (Table 4). Only PBPM had an amount of cysteic and methionine sulfoxide higher than analytical detection threshold (50 mg/kg) with 133 and 105 mg/kg respectively. Moreover, a greater non-reactive lysine was determined for the PBPM and the diet PBPM.

Table 4: Non-reactive lysine, cysteic acid, methionine sulfone, methionine sulfoxide of the experimental ingredients and diets

| | Non-reactive lysine (% of total lysine) | Cysteic acid (mg/kg) | Methionine sulfone (mg/kg) | Methionine sulfoxide (mg/kg) |
|---|---|---|---|---|
| CC | 16.7 | <50 | <50 | <50 |
| PBPM | 18.0 | 133 | <50 | 105 |
| CC diet | 14.8 | <50 | <50 | <50 |

(continued)

| | Non-reactive lysine (% of total lysine) | Cysteic acid (mg/kg) | Methionine sulfone (mg/kg) | Methionine sulfoxide (mg/kg) |
|---|---|---|---|---|
| PBPM diet | 16.0 | <50 | <50 | <50 |

*In vitro* Protein Digestibility: The analysis of the experimental ingredients showed a greater ileal *in vitro* protein digestibility for the CC by 4.1% (Table 5).

Table 5: *In vitro* protein digestibility of the experimental ingredients

| | *In vitro* protein digestibility (%) |
|---|---|
| CC | 93.1 |
| PBPM | 89.0 |

Apparent total tract digestibility: In the dog assay, no differences were observed in apparent total tract digestibility for dry matter (DM), organic matter (OM), Ash, and gross energy (GE). Apparent total tract digestibility was significantly different in CP (p-value = 0.037) and in fat (p-value = 0.001) between the two experimental diets with higher values for CC group vs. PBPM group (Table 6).

No difference was seen between both diets for the apparent total tract digestibility of the amino acids. However, it was a trend that leucine (p-value = 0.085) and glycine (p-value = 0.070) plasma concentrations were different between the two groups of dogs.

Corrected amino acids digestibilities were calculated (Table 7).

AA absorption test: All dog EEAs, except cysteine, were measured for kinetic of amino acid absorption. Most profiles were identical after both diets with a quick rise after the meal and a slow progressive return to baseline level in post-absorptive state. Lysine was the only amino acid that decreased post-prandially below baseline level. **FIGS. 1A** and **1B** are graphs showing plasmatic concentration of lysine and arginine over time, respectively. Values are mean+SD for 12 dogs.

Time had a significant effect on plasma amino acid concentration for all AA and for the ratio Lys/TEAA. For a time period, when a significant difference was shown between the two diets, the p-value corresponding was noticed.

Table 6: Apparent total tract proximates and amino acids digestibilities of the experimental diets (%)

| | CC diet | PBPM diet | p-value |
|---|---|---|---|
| DM | 84.96 ± 1.42 | 84.96 ± 1.6 | 0.999 |
| OM | 89.00 ± 1.13 | 88.61 ± 1.28 | 0.532 |
| CP | 88.14 ± 1.68 | 86.10 ± 1.85 | 0.037 |
| Fat | 96.59 ± 0.97 | 94.35 ± 1.07 | 0.001 |
| Ash | 35.21 ± 5.44 | 37.97 ± 6.56 | 0.377 |
| GE | 89.25 ± 1.12 | 88.42 ± 1.23 | 0.182 |
| Corrected CP ileal digestibility[1] | 84.06 ± 3.43 | 79.91 ± 3.77 | 0.037 |

[1]Corrected CP ileal digestibility using the equation developed by Hendrik, et al. (2012)

$$\text{Corrected Ileal CP/AA digestibility} = \frac{\text{Apparent total tract CP/AA digestibility} - 46.869}{0.491}$$

Table 7: Area under the curve of the amino acid and associated p-value

| | | Area Under the Curve (mean ± SEM) | Diet[2] (p-value) | Period (p-value) | Interaction Diet*Group (p-value) |
|---|---|---|---|---|---|
| Arg | CC | 511 ± 23 | 0.012 | 0.987 | 0.845 |

(continued)

| | | Area Under the Curve (mean ± SEM) | Diet[2] (p-value) | Period (p-value) | Interaction Diet*Group (p-value) |
|---|---|---|---|---|---|
| | PBPM | 468 ± 23 | | | |
| His | CC | 358 ± 8 | 0.891 | 0.416 | 0.774 |
| | PBPM | 357 ± 12 | | | |
| Ile | CC | 161 ± 6 | 0.132 | 0.902 | 0.576 |
| | PBPM | 173 ± 9 | | | |
| Leu | CC | 552 ± 21 | 0.028 | 0.908 | 0.759 |
| | PBPM | 597 ± 30 | | | |
| Lys | CC | 371 ± 19 | 0.159 | 0.628 | 0.044 |
| | PBPM | 347 ± 22 | | | |
| Met | CC | 203 ± 9 | 0.300 | 0.944 | 0.556 |
| | PBPM | 209 ± 13 | | | |
| Phe | CC | 243 ± 7 | 0.564 | 0.721 | 0.817 |
| | PBPM | 247 ± 11 | | | |
| Thr | CC | 284 ± 11 | 0.617 | 0.815 | 0.469 |
| | PBPM | 284 ± 14 | | | |
| Trp | CC | 261 ± 24 | 0.279 | 0.001 | 0.095 |
| | PBPM | 275 ± 23 | | | |
| Tyr | CC | 284 ± 11 | 0.975 | 0.687 | 0.031 |
| | PBPM | 284 ± 14 | | | |
| Val | CC | 399 ± 13 | 0.170 | 0.695 | 0 .585 |
| | PBPM | 416 ± 19 | | | |
| TEAAs | CC | 3634 ± 94 | 0.699 | 0.590 | 0.803 |
| | PBPM | 3662 ± 142 | | | |
| Ratio Lys/ TEAAs | CC | 2.34 ± 0.14 | 0.538 | 0.384 | 0.904 |

[2] Analysis of variance, with AUC as the dependent variable and diet, group and the interaction diet*group as the independent variables.

The area under the curve had a significant effect on the absorption of arginine and leucine. Group and diet had a significant interaction effect on lysine, tyrosine and the ratio of Lys/TEEAs.

Discussion

Chemical characterization

The source of raw materials (heads, feet, entrails) is the major factor affecting CC and PBPM quality. Hydroxyproline is the main component of collagen that is characteristic to be not sensitive to trypsin hydrolysis, preventing the unfolding of the protein and decreasing accessibility of other enzymes. In the study, 12.2% of the CP fraction was collagen for the CC diet against 10.0% for the PBPM diet. However, CC *in vitro* digestibility was 4.1% higher than PBPM with an *in vitro* CP digestibility of 93.1% vs. 89.0%. Therefore, collagen may not be an accurate factor to measure the quality of a protein source.

Experimental ingredients differed in amount of oxidized sulfur amino acid. Oxidized amino acids are of importance to estimate protein quality; for example, methionine sulfone and cysteic acid cannot be utilized by the weanling rat, but methionine sulfoxide can be partially utilized. PBPM had a greater cysteic acid and methionine sulfoxide level, which may be explained by the high temperature using in the rendering process. However, that difference was not measured in the diets because cysteic acid, methionine sulfone, and methionine sulfoxide were all below 50 mg/kg.

Non-reactive lysine determinations were made on the experimental diet to see whether the protein source may differ in term of quality; reactive lysine is important for accurate determination of lysine digestibility in moist and dry cat foods. Results found that both ingredients have about the same proportion of non-reactive lysine, which reveals that the rendering process which produces meal may not be as strong as expected. PBPM are ground and heated for about 30 to 40 minutes to a temperature of 120 to 140°C. The products are then dried and ground again into fine particles to make the PBPM. The process is therefore strong in terms of temperature and duration, which may induce more easily the reaction of the $\varepsilon$-amino group of lysine by the Maillard reaction. However, the low percentage of reducing sugar contained in the protein source incorporated may have limited this reaction.

The CC process is first a cooking step of the CC frozen for about 80°C for only 12 min. Then, the meat slurry is ground at 70°C to be included into the extruder. Therefore, although the temperature and the time needed for the CC process is lower than the PBPM process, the CC diet did not contain a lower non-reactive lysine content.

Dog Assay

Digestibility results were determined between the two groups of 8 different dogs from the first period.

Apparent total tract proximates digestibility: Diets were formulated to target a percentage of CP and fat of 18% and 9% respectively. On DM basis, both experimental diets had an identical amount of crude protein and were also isoenergetic. As targeted, experimental ingredients contributed to 25% of the overall crude proteins. To reach this proportion, CP content was around 18% in both diets, which is close to the minimum amount recommended by FEDIAF. The low proportion of dietary protein may have contributed to show limiting amino acids.

Apparent digestibilities of CP and fat of the CC diet were significantly higher than the PBP meal.

For net uptake, an example was made with a dog eating 1000 Kcal a day and weighing 15 kg. The calculations was made as shown in Table 9, following the below formula:

$$\text{Average uptake (g of CP)} = \text{(Dietary amount of CP)*(GE)*(net CP intake to eat 1000 Kcal)*(Corrected ileal N digestibility)}$$

$$\text{Uptake of CP by metabolic weight} = \frac{\text{(Average uptake)}}{\text{(Metabolic weight of a 15 kg dog)}}$$

$$\text{CP available after removal of the mean oxidation} = \text{Uptake of CP by metabolic weight} - \text{Mean oxidation}$$

Table 8: Net uptake calculation

| Diet | Dietary amount of CP (% DM) | GE (Kcal/kg) | net CP intake to eat 1000 Kcal (g of CP) | Corrected ileal N digestibility (%) | avg. uptake (g of CP) | Metabolic weight of a 15 kg dog (kg $BW^{\wedge 0,75}$) | Uptake of CP by metabolic weight (g CP/kg $BW^{\wedge 0,75}$) | CP available after removal of min body protein oxidation (Humbert 2001) (3.93 g CP/kg $BW^{\wedge 0,75}$ per day) |
|---|---|---|---|---|---|---|---|---|
| CC diet | 18.87 | 3.48 | 54.22 | 84.07 | 45.59 | 7.62 | 5.98 | 2.05 |
| PBPM diet | 18.03 | 3.36 | 53.66 | 79.92 | 42.88 | 7.62 | 5.63 | 1.70 |

Humbert et al. (2001) reported a value of 3.93 g/kg $BW^{\wedge 0.75}$ per day for body protein oxidation. Both diets exceeded this basal protein oxidation, with 5.98 and 5.63 g/kg $BW^{\wedge 0.75}$ for CC and PBPM diets respectively. However, the CC diet provided 20% more protein above this minimum requirement vs. the PBPM diet (2.05 vs. 1.7 g/kg $BW^{\wedge 0.75}$

respectively). The difference may be small, knowing that both diets already meet minimum requirement, but may represent a nutritional advantage on a longer term for lean body mass maintenance, especially in animals with higher needs per metabolic body weight like growing animals, pregnant/lactating female dogs, or working dogs.

Amino acid absorption test: The AA levels were strongly affected by the postprandial sampling time. All amino acids concentration increased postprandially with a peak between 3 and 5 hours after the meal. The postprandial kinetics of amino acid absorption showed a marked increase in the vast majority of essential amino acids during the 2-3 first hours during the postprandial state, reflecting the good protein digestibility observed in both diets.

However, for lysine, no increase above baseline was observed indicating that lysine was the most limiting amino acid in the diet (**FIG. 1A**). This observation is not surprising as both diets were formulated close to the minimum recommended allowance advised by FEDIAF. Lysine is very sensitive to thermal processes which reduce its availability by the formation of non-available Maillard compounds. This result reinforces the fact that protein quality is key to ensure that lysine requirements are met at lower protein intakes. Even if not significant, there was a tendency for a higher area under the curve in the CC diet indicating that lysine was less limiting than in the PBPM diet.

Another amino acid known as "limiting" in the literature showed significant differences between the two diets: arginine enrichment was significantly higher with the CC diet vs. PBPM diet (**FIG. 1B**). Dogs fed the CC diet showed higher plasma arginine values overtime that were significant 1 hour and 2 hours after the meal ingestion, indicating a better availability of arginine in the CC diet. The overall AUC for arginine was also significantly higher in CC vs. PBPM diet. This finding is of particular interest as arginine is considered as a key amino acid in urea cycle and protein synthesis pathways. Arginine is an intermediate component of the urea cycle and is known as an allosteric activator of the urea desintoxification. Also, arginine is well known as an anabolic amino acid by its secretagogue action on hormones like insulin and growth hormone. This better arginine status together with better lysine ileal digestibility could lead to a higher capacity for protein synthesis during the postprandial state.

Conclusion

Overall, the results of the analysis and the trials conducted in the study were able to give a better estimation of the relative dietary protein quality of both experimental diets. As the apparent total tract CP digestibility of CC diet revealed to be significantly higher than the PBPM diet, protein quality of CC may be better than the PBPM. The higher *in vitro* protein digestibility of the CC may indicate a higher ileal digestibility and therefore may explain the observed difference in total tract CP digestibility.

The amino acid absorption test showed that lysine was the first limiting amino acid and revealed a trend for a higher area under the curve for lysine in dogs fed the CC diet. This difference was even more sizable for arginine, another key limiting amino acid in protein metabolism and synthesis. Arginine was more available with CC diet as shown by a significantly higher plasma status.

Therefore, in conclusion, dietary CC proteins may have a higher bioavailability than dietary PBPM proteins.

EXAMPLE 2

Based on the results of the study in Example 1, a second study was conducted to verify if the differences in digestibilities and blood enrichments could also be observed between products with higher fat and protein contents (25% protein, 15% fat for 100 grams of final product).

Three diets were produced with different amount of real meat in their recipe. The first diet contained 20% of real meat, the second diet contained 14% of real meat (14% being the minimal amount of real meat in the recipe that allows the claim of "rich in real meat"), and the third diet contained no real meat. The three diets contained meat meal as a source of animal protein. The diets were formulated to meet or exceed the minimum requirements for adult dogs established by FEDIAF. The diets were formulated to contain the same proportion, on a dry matter (DM) basis, of crude protein (CP), fat, crude fiber, carbohydrates (CHO), and ash. The only difference between the three diets was the amount of real meat in the source of animal protein.

Table 1: Composition of the three diets

| Diet | 1 | 2 | 3 |
|---|---|---|---|
| % of real meat | 20% | 14% | 0% |
| Primary ingredient | chicken | poultry meal | poultry meal |
| Dry matter (%) | 92.5 | 92.4 | 92.2 |
| Moisture (%) | 7.5 | 7.6 | 7.8 |

(continued)

| Diet | 1 | 2 | 3 |
|---|---|---|---|
| Protein (%) | 25.3 | 25.2 | 25.2 |
| Protein from real meat in 100 g of product (%) | 3.9 | 2.5 | 0.0 |
| Contribution of real meat to total protein (%) | 15.5 | 10.1 | 0.0 |
| Fat (%) | 14.9 | 14.9 | 15.0 |
| Ash (%) | 7.5 | 7.4 | 7.2 |
| Crude fiber (%) | 2.1 | 2.1 | 2.1 |
| Carbohydrates (%) | 42.7 | 42.9 | 42.7 |

Twelve dogs were used for the study with various breeds, gender and sterilized status. Dogs were individually housed in indoor kennels with continual free access to a large external court. Diets were given in one single portion at 9:00. Dogs had continuous access to water. All dogs were considered healthy based on results of physical examination and clinical laboratory tests.

The twelve dogs were split into three groups of four dogs with similar age and breed distribution. The study was a cross-over study of 30 days divided in three periods of ten days, thus at the end of the study, each dog has tested each of the three diets.

Table 2: Cross-over study design

| Group | Period 1 | Period 2 | Period 3 |
|---|---|---|---|
| Group 1 | Diet 1 | Diet 2 | Diet 3 |
| Group 2 | Diet 2 | Diet 3 | Diet 1 |
| Group 3 | Diet 3 | Diet 1 | Diet 2 |

The 10 day period was divided into two parts; the first four days being the adaptation part when only daily food consumption and fecal consistency were assessed, and the six remaining days being the test part during which besides the assessment of daily food consumption and fecal consistency, feces were collected, weighed and stored at -20°C. Amino acid blood enrichment following meal was also assessed.

Diets were analyzed for complete AA and fatty acid profiles. Diet and feces were analyzed for moisture, CP, crude fat, crude fiber, ash, and GE.
Total tract nutrient digestibilities were calculated as:

$$[\text{nutrient intake (total g/7 d) - nutrient output (total g/7 d)}]/\text{nutrient intake (total g/7 d)}$$

Net uptake for linoleic fatty acid was calculated as:

$$[\text{daily food intake (total g/7 d)*concentration of fatty acid in diet (\%)*digestibility of fatty acid (\%)*contribution of linoleic fatty acid to total fatty acid profile (\%)}]$$

Blood samples (5 mL) were collected through the cephalic catheter into two 2.5-mL syringes using a 1" x 20 g needle. Immediately prior to collecting the 5 mL blood samples, 0.5 mL of blood was collected and discarded to remove residual anticoagulant in the catheter. Blood was collected 0.5 hour before meal was given and at 1, 2, 3, 5 h after meal was eaten. Following collection, half of the blood was immediately transferred into a 5-mL Na-heparin tube the other half was transferred to a 3-mL dry tube. In total, per period, 20 mL (2.35% of total blood volume) of blood was collected, which is below the GIRCOR recommendations.

Blood was centrifuged at 3,000 x g for 10 min at room temperature in a clinical centrifuge. The plasma and the serum obtained respectively from the Na-heparin tubes and the dry tubes were extracted and then stored at -80°C analysis.

AA concentrations (Lysine, Leucine, Methionine, Arginine, Glutamine) were performed on plasma samples with Biochrom amino acid analysers using classical ion-exchange liquid chromatography with post-column Ninhydrin derivatisation and photometric detection. Areas under the curve for each amino acid and diet were calculated using trapezoidal rule and were adjusted with the basal value.

Paired T-tests were performed for crude protein, CHO, fat, ash, GE digestibilities between each diet. Analysis of Variance (ANOVA) was performed for insulinemia and AA concentration to test the effect of time, diet, period, group and interaction between diet and group. For each amino acid, areas under the curve were compared using paired T-test.

Results and discussion

Diet analysis: Diets were formulated to contain the same proportion of crude protein and fat, however they were formulated with different amount of real meat (20%, 14% or 0%) leading to a difference in fatty acid profile between the three diets (**FIG. 2**). Increasing the concentration of real meat in the kibbles leads to a higher contribution of PUFAs to total fatty acid profile to the expense of SFAs. If the contribution of linoleic and stearic fatty acids to the total fatty acid profile are totaled, the same result for the three diets is found. This clearly proves that rendering process converts linoleic acid into its main hydrogenated form: stearic fatty acid.

As shown in **FIG. 3**, there was a very high similarity for amino acid profiles between each diet. This finding can be explained by the contribution of real meat to total protein content in the three diets respectively 15.5, 10.6 and 0%.

As shown in **FIG. 4**, high digestibilities of CP, Fat, CHO and GE were observed for the three diets, proving the high quality of the three diets. No significant difference was observed between each of the three diets concerning digestibility for CP. However, a trend of increased CP digestibility was observed with an increase of the amount of real meat in the diet. This increase was even higher when using the equation of Hendricks predicting ileal digestibility.

No significant difference was observed between each of the three diets concerning digestibility for fat. In this study, the increase of saturated fatty acids (stearic fatty acid) in the diet without real meat did not affect negatively fat digestibility, likely due to the low difference of fatty acid profiles between the three diets. No significant difference was observed between each of the three diets concerning digestibility for CHO, ash and GE.

As shown in **FIG. 5**, net uptake for linoleic fatty acids was significantly higher in diets made with real meat compared to the diet without real meat. This result is logical since diets made with real meat show higher concentration of linoleic fatty acid.

For the three diets, amino acids concentrations showed marked increases during postprandial period, confirming the good protein digestibility observed. Nevertheless, no effect of diets on blood concentration for the different amino acids was observed.

The comparison of the results from the first study (Example 1) using diets containing 18% of protein and the ones of this study using diets containing 25% of protein brought valuable results on the benefits of increasing protein content in the diet. Indeed, as shown in the graphs in **FIGS. 6** and **7**, very different patterns of enrichment between diets from the two studies can be observed. Dogs fed with the diets with 25% of protein showed higher blood enrichment in methionine, lysine and arginine confirming that a higher protein uptake leads to a higher AA absorption. However, blood enrichment in AA was not directly correlated to the level of AA supply. Indeed, in dogs fed diets with the diets with 18% of protein, curves show only slight increases for arginine and methionine and a decrease for lysine whereas in dogs fed diets with 25% of protein, curves show strong increases. This finding can be explained by the fact that the AA supply is lower in dogs fed with the diets with 18% of protein compared to dogs fed with diets with 25% of protein while the demand for these indispensable amino acids in post-prandial period might be of the same amplitude. Therefore, in the case of the diets with 18% of protein, this demand for protein synthesis that draws on amino acid pool is either barely compensated by the supply in the case of methionine and arginine or not compensated by the supply in the case of lysine.

The period in which the second study was conducted coincided with a period of extreme cold weather in the area in which the study was conducted. Due to these extreme weather conditions, the dogs had difficulty in maintaining body weight. A better body weight maintenance was observed in the dogs fed the 14% and 20% chicken compared to 0% chicken (**FIG. 8**).

Conclusion

The observations relating to a higher digestibility for protein and fat in kibbles made with real meat from the first study (Example 1) were not as significant in this study; this is probably due to the lower contribution of real meat to the total fat and protein contents. However, this study showed that the use of real meat allows higher uptake of linoleic fatty acid promoting potentially health benefits.

The comparison of the results from the two first studies show that the increase of protein content in kibbles allows to compensate widely the demand in the amino acid for protein synthesis in post prandial period, thus feeding dogs with kibbles containing 25% of protein may ensure a higher protein synthesis in postprandial period and a better maintenance of muscle mass in long-term.

EXAMPLE 3

Results of the first two studies (Examples 1 and 2) gave insights to an increase of overall protein and fat qualities along with an increase of real meat content in the kibbles. This increase of quality leads to higher digestibilities and higher utilization of amino acids for protein synthesis in postprandial period.

The aim of the third study was to assess if the higher digestibilities of protein and fat and linoleic fatty acid content of diet made with real meat could provide health benefits during two very high demanding periods: gestation and lactation period in female dogs.

The study was performed on gestating and lactating female dogs in breeders using two diets; one diet containing real meat as a source of animal protein, the other diet containing only meat meal as a source of animal protein. Health parameters measuring indirectly the quality of the diet were used.

Two diets were produced in the study, with different amount of real meat in their recipe. The first diet (Diet A) contained 14% of real meat for 100 g of product, the second diet (Diet B) contained no real meat.

Table 1: Composition of Diets

| Diet | 1 | 2 |
|---|---|---|
| % of real meat | 14% | 0% |
| Primary ingredient | poultry meal | poultry meal |
| Dry matter (%) | 92.0 | 92.0 |
| Moisture (%) | 8.0 | 8.0 |
| Protein (%) | 29.0 | 29.0 |
| Protein from real meat in 100 g of product (%) | 2.5 | 0.0 |
| Contribution of real meat to total protein (%) | 8.7 | 0.0 |
| Fat (%) | 17.0 | 17.0 |
| Ash (%) | 6.9 | 6.9 |
| Crude fiber (%) | 1.7 | 1.7 |
| Carbohydrates (%) | 37.5 | 37.5 |

Diets were formulated to meet or exceed the minimum requirements for gestation, lactation and growth periods established by FEDIAF guidelines. Diets were formulated to contain the same amounts, on a DM basis, of CP, fat, crude fiber, carbohydrates (CHO), and ash.

Twelve female dogs were included in the study with various breeds (French Bulldog, Carlin, English Bulldog, Labrador, Fox Terrier). The female dogs used in the study came from breeders. They were more than one year of age and were in their second heat period.

The test design was based on a protocol of The Association of American Feed Control Officials (AAFCO) to prove a gestation/lactation claim. The twelve female dogs were split into two groups with similar age and breed distribution; each group was fed with one of the two diets from the fifth week of gestation to the end of the first month of lactation for a total duration of two months. The diet was the sole source of food during the whole test.

Diets were analyzed for complete AA and fatty acid profiles using oxidative hydrolysis and an amino-acid analyzer, respectively.

Breeders were asked to follow on a daily basis the food consumption of their female dogs, and food ration was calculated to fulfill energy needs. Breeders were asked to follow on a weekly basis the evolution of body weight and fecal consistency of their female dogs, to do so they were given a four point scale. After parturition, breeders were asked to follow on a weekly basis the evolution of body weight of the puppies in the litter.

Main parameters assessed were: (a) BW evolution (female dogs and puppies), (b) food intake (female dogs), (c) food efficiency on puppies' growth during lactation, and (d) milk composition (protein, AAs).

Table 1: Diet analysis

| | Diet A (14% slurry) | Diet B (0% slurry) |
|---|---|---|
| Moisture | 7.4 | 7.4 |

(continued)

| | Diet A (14% slurry) | Diet B (0% slurry) |
|---|---|---|
| Protein | 30.3 | 30.2 |
| Fat | 16.9 | 16.6 |
| Linoleic Acid | 2.4 | 2.1 |

As shown in **FIG. 9**, no significant differences were observed in product digestibility between diets.

Animals: 5 female dogs completed the trial (2 French Bulldogs, 1 Border Collie, 1 Labrador, 1 Bearded Tchec dog). The litter size of the Diet A group was 11 puppies (French Bulldog: 7 puppies, Border Collie: 4 puppies). The litter size of the diet B group was 14 puppies (French Bulldog: 4 puppies, Labrador: 9 puppies, Tchec Bearded Dog: 1 puppy).

The mothers' body weight (BW) evolution during late gestation is shown in **FIGS. 10A** and **10B.** The Tchec Bearded dog was not included in the treatment of data as the litter size was considered too low (1 puppy) and not enough "challenging" in terms of body weight maintenance considering breed size (large dog). Female dogs fed with Diet A tended to better cope with late gestation and lactation periods in terms of weight maintenance (e.g., **FIG. 11**).

As shown in **FIGS. 12A** and **12B**, the puppies' growth rate during lactation was higher on Diet A. As shown in **FIG. 13**, Diet A was more efficient to promote weight gain, especially during the late lactation period, when the puppies' body weight gain was normalized with mother's food intake.

Diet A was more efficient at promoting growth in puppies during lactation. There was also a trend for better weight maintenance of the pregnant/lactating female dogs fed Diet A. This result can be due to higher level of essential fatty acids coming from the slurry, but availability of amino acids probably played a role too.

These results need to be considered cautiously given the limited number of female dogs involved and the variety of breeds and body weight. However, when considering only the two French Bulldogs from the same breeder, the same differences were observed (**FIGS. 14A** and **14B**).

Sensory analysis of the milk from the lactating female dogs was done by breeders using three parameters: translucidity, quantity, and overall quality (**FIGS. 15A-15C** and **16**). Milk quality was better rated by breeders for lactating female dogs fed with Diet A.

To determine the milk protein content in the lactating female dogs, milk was sampled by breeders at three different periods (first week, middle and late lactation). As shown in **FIG. 17**, there was higher protein content in lactating female dogs receiving Diet A.

When focusing only on the two French Bulldogs from the same breeding house, a clear difference in milk protein content was observed. As shown in **FIG. 18**, the lactating female French Bulldog on Diet A produced milk with a high level of protein while the lactating female French Bulldog on Diet B produced a milk with a protein content in-line with published data in which ten lactating Beagle dogs produced a milk protein range of 6.7 - 9.6% throughout lactation. This higher protein content in French Bulldogs receiving Diet A could explain the higher growth rate of the puppies.

As shown in **FIG. 19**, the average milk amino acids profile in female dogs fed with Diet A was significantly different from the profile in female dogs fed with Diet B, several amino acids being in higher levels in female dogs fed with 14% slurry. Correlation between amino acid pattern in milk vs. diet is an indirect sign of diet amino acids availability. As shown in **FIGS. 20A-20C**, the correlation between milk and Diet A was high (0.94 to 0.96) at the different lactation periods, indicating a high availability of the amino acids coming from the diet. For similar dietary amino acid profile, Diet A was more efficient than Diet B to promote a high quality milk as demonstrated by the higher milk amino acid content for a same dietary amino acid content. These correlations confirm the results from the first study (Example 1) showing a higher quality of protein in slurry compared to poultry meal and a better availability of amino acids.

Conclusion

This third study confirmed that dog food formulated with meat slurry has a better nutritional performance than a diet formulated without slurry. During a challenging long term feeding trial, the slurry-based diet showed promoted good performance in terms of milk production quantity and quality, body weight maintenance in the lactating female dog, and good growth rate of suckling puppies. These effects are most probably due to a high availability of both linoleic acid and essential amino acids.

## Claims

**1.** A method of producing a dry pet food composition, the method comprising:

forming a meat slurry from fresh or frozen meat;
adding the meat slurry to a dry blend of ingredients to form a mixture in which the meat slurry is 14 wt% to 20 wt% of the mixture;
subjecting the mixture to extrusion cooking to form an extrudate; and
processing the extrudate to form the pet food composition, wherein processing the extrudate comprises cutting the extrudate into pieces and drying the pieces.

**2.** The method of Claim 1, wherein the forming of the meat slurry comprises subjecting the fresh or frozen meat to size reduction and cooking the size-reduced fresh or frozen meat with direct steam injection at a temperature of about 71 °C or less.

**3.** The method of Claim 2, wherein the forming of the meat slurry comprises emulsifying the steam-injected meat.

**4.** The method of Claim 1, wherein the mixture comprises about 18% to about 30% protein.

**5.** The method of Claim 1, wherein the dry blend comprises a whole grain.

**6.** The method of Claim 1, wherein the dry blend comprises a fiber.

**7.** The method of Claim 1, wherein the meat slurry is added to the dry blend of ingredients in a preconditioner, and the mixture is fed from the preconditioner to an extruder that performs the extrusion cooking.

**8.** The method of Claim 7, wherein the extrusion cooking is performed in the extruder at a temperature of 105 to 130 °C at a pressure of 1724 to 3447 kPa (250 to 500 psi) for a time period less than 40 seconds.

**9.** The method of Claim 1, wherein the drying of the pieces reduces a moisture content of the pieces to about 6% to about 9% moisture.

**10.** The method of Claim 9, wherein the processing of the extrudate comprises coating the dried pieces with at least one of an animal fat or an animal digest.

**11.** A dry pet food composition comprising 14 wt% to 20 wt% of meat that is not meat meal, wherein the meat that is not meat meal is a meat slurry from fresh or frozen meat.

**12.** The dry pet food composition of Claim 11, having a protein content of about 18% to about 30%.

**13.** The dry pet food composition of Claim 12, wherein the dry pet food composition comprises the meat slurry and a protein source selected from the group consisting of a vegetable protein, a meat meal, and combinations thereof.

**14.** The dry pet food composition of Claim 11, wherein the meat that is not meat meal is provided by making the dry pet food composition with a process comprising subjecting fresh or frozen meat to size reduction, cooking the size-reduced fresh or frozen meat with direct steam injection, emulsifying the steam-injected meat to form a slurry, and adding the slurry to one or more other ingredients.

**15.** A method of improving bioavailability of at least one of an essential fatty acid or an essential amino acid relative to an initial dry pet food formulation comprising a meat meal that provides at least a portion of a protein content of the initial dry pet food formulation, the method comprising:

adjusting the initial dry pet food formulation to replace at least a portion of the meat meal with meat provided by meat that is not meat meal, wherein the meat that is not meat meal is a meat slurry from fresh or frozen meat, wherein the meat slurry is 14 wt% to 20 wt% of the adjusted dry pet food formulation, wherein the adjusted dry pet food formulation has a protein content that is about equal to the protein content of the initial dry pet food formulation; and
producing a kibble according to the adjusted dry pet food formulation.

**16.** The method of Claim 15, wherein the initial dry pet food formulation comprises non-meat ingredients, and the adjusted dry pet food formulation has the same amount of the non-meat ingredients relative to the initial dry pet food formulation.

**17.** The method of claim 15 wherein the essential fatty acid is linoleic acid or the essential amino acid is arginine or lysine.

**Patentansprüche**

**1.** Verfahren zum Herstellen einer trockenen Haustierfutterzusammensetzung, wobei das Verfahren umfasst: das Bilden eines Fleischbreis aus frischem oder gefrorenem Fleisch;
das Hinzufügen des Fleischbreis zu einem Trockengemisch von Bestandteilen, um eine Mischung zu bilden, in der der Fleischbrei 14 Gew.-% bis 20 Gew.-% der Mischung ausmacht;
das Unterziehen der Mischung einem Extrusionskochen, um ein Extrudat zu bilden; und
das Verarbeiten des Extrudats, um die Haustierfutterzusammensetzung zu bilden, wobei das Verarbeiten des Extrudats das Zerschneiden des Extrudats in Stücke und das Trocknen der Stücke umfasst.

**2.** Verfahren nach Anspruch 1, wobei das Bilden des Fleischbreis das Unterziehen des frischen oder gefrorenen Fleisches einer Größenreduzierung und das Kochen des größenreduzierten frischen oder gefrorenen Fleisches mit direkter Dampfeinspritzung bei einer Temperatur von etwa 71 °C oder weniger umfasst.

**3.** Verfahren nach Anspruch 2, wobei das Bilden des Fleischbreis das Emulgieren des dampfeingespritzten Fleisches umfasst.

**4.** Verfahren nach Anspruch 1, wobei die Mischung etwa 18 % bis etwa 30 % Protein umfasst.

**5.** Verfahren nach Anspruch 1, wobei das Trockengemisch ein Vollkorn umfasst.

**6.** Verfahren nach Anspruch 1, wobei das Trockengemisch eine Faser umfasst.

**7.** Verfahren nach Anspruch 1, wobei der Fleischbrei dem Trockengemisch von Bestandteilen in einem Vorkonditionierer hinzugefügt wird und die Mischung aus dem Vorkonditionierer einem Extruder zugeführt wird, der das Extrusionskochen durchführt.

**8.** Verfahren nach Anspruch 7, wobei das Extrusionskochen in dem Extruder bei einer Temperatur von 105 bis 130 °C bei einem Druck von 1.724 bis 3.447 kPa (250 bis 500 psi) für einer Zeitraum von weniger als 40 Sekunden durchgeführt wird.

**9.** Verfahren nach Anspruch 1, wobei das Trocknen der Stücke einen Feuchtigkeitsgehalt der Stücke auf etwa 6 % bis etwa 9 % Feuchtigkeit verringert.

**10.** Verfahren nach Anspruch 9, wobei das Verarbeiten des Extrudats das Beschichten der getrockneten Stücke mit mindestens einem von einem tierischen Fett oder einem tierischen Extrakt umfasst.

**11.** Trockene Haustierfutterzusammensetzung, umfassend 14 Gew.-% bis 20 Gew.-% Fleisch, das kein Fleischmehl ist, wobei das Fleisch, das kein Fleischmehl ist, ein Fleischbrei aus frischem oder gefrorenem Fleisch ist.

**12.** Trockene Haustierfutterzusammensetzung nach Anspruch 11 mit einem Proteingehalt von etwa 18 % bis etwa 30 %.

**13.** Trockene Haustierfutterzusammensetzung nach Anspruch 12, wobei die trockene Haustierfutterzusammensetzung den Fleischbrei und eine Proteinquelle umfasst, die ausgewählt ist aus der Gruppe, bestehend aus einem pflanzlichen Protein, einem Fleischmehl und Kombinationen davon.

**14.** Trockene Haustierfutterzusammensetzung nach Anspruch 11, wobei das Fleisch, das kein Fleischmehl ist, bereitgestellt wird, indem die trockene Haustierfutterzusammensetzung mit einem Verfahren hergestellt wird, umfassend das Unterziehen von frischem oder gefrorenem Fleisch einer Größenreduzierung, das Kochen des größenreduzierten frischen oder gefrorenen Fleisches mit direkter Dampfeinspritzung, das Emulgieren des dampfeingespritzten Fleisches, um einen Brei zu bilden, und das Hinzufügen des Breis zu einem oder mehreren weiteren Bestandteilen.

**15.** Verfahren zum Verbessern der Bioverfügbarkeit von mindestens einem von einer essentiellen Fettsäure oder einer essentiellen Aminosäure relativ zu einer anfänglichen trockenen Haustierfutterformulierung, die ein Fleischmehl umfasst, das mindestens einen Teil eines Proteingehalts der anfänglichen trockenen Haustierfutterformulierung bereitstellt, wobei das Verfahren umfasst:

das Anpassen der anfänglichen trockenen Haustierfutterformulierung, um mindestens einen Teil des Fleischmehls durch Fleisch zu ersetzen, das von Fleisch bereitgestellt wird, das kein Fleischmehl ist, wobei das Fleisch, das kein Fleischmehl ist, ein Fleischbrei aus frischem oder gefrorenem Fleisch ist, wobei der Fleischbrei 14 Gew.-% bis 20 Gew.-% der angepassten trockenen Haustierfutterformulierung ausmacht, wobei die angepasste trockene Haustierfutterformulierung einen Proteingehalt aufweist, der etwa gleich dem Proteingehalt der anfänglichen trockenen Haustierfutterformulierung ist; und
das Herstellen eines Trockenfutters gemäß der angepassten trockenen Haustierfutterformulierung.

**16.** Verfahren nach Anspruch 15, wobei die anfängliche trockene Haustierfutterformulierung Nicht-Fleischbestandteile umfasst und die angepasste trockene Haustierfutterformulierung die gleiche Menge an Nicht-Fleischbestandteilen relativ zu der anfänglichen trockenen Haustierfutterformulierung aufweist.

**17.** Verfahren nach Anspruch 15, wobei die essentielle Fettsäure Linolsäure ist oder die essentielle Aminosäure Arginin oder Lysin ist.

## Revendications

**1.** Procédé de production d'une composition alimentaire sèche pour animal de compagnie, le procédé comprenant :
la formation d'une bouillie de viande à partir de viande fraîche ou congelée ;
l'ajout de la bouillie de viande à une combinaison sèche d'ingrédients pour former un mélange dans lequel la bouillie de viande représente 14 % en poids à 20 % en poids du mélange ;
la soumission du mélange à une cuisson-extrusion pour former un extrudat ; et
le traitement de l'extrudat pour former la composition alimentaire pour animal de compagnie, dans lequel le traitement de l'extrudat comprend la découpe de l'extrudat en morceaux et le séchage des morceaux.

**2.** Procédé selon la revendication 1, dans lequel la formation de la bouillie de viande comprend la soumission de la viande fraîche ou congelée à une réduction de taille et la cuisson de la viande fraîche ou congelée réduite en taille avec une injection directe de vapeur à une température d'environ 71 °C ou moins.

**3.** Procédé selon la revendication 2, dans lequel la formation de la bouillie de viande comprend l'émulsification de la viande injectée de vapeur.

**4.** Procédé selon la revendication 1, dans lequel le mélange comprend environ 18 % à environ 30 % de protéine.

**5.** Procédé selon la revendication 1, dans lequel la combinaison sèche comprend une céréale complète.

**6.** Procédé selon la revendication 1, dans lequel la combinaison sèche comprend une fibre.

**7.** Procédé selon la revendication 1, dans lequel la bouillie de viande est ajoutée à la combinaison sèche d'ingrédients dans un préconditionneur, et le mélange est alimenté du préconditionneur à une extrudeuse qui met en oeuvre la cuisson-extrusion.

**8.** Procédé selon la revendication 7, dans lequel la cuisson-extrusion est mise en oeuvre dans l'extrudeuse à une température de 105 à 130 °C à une pression de 1724 à 3447 kPa (250 à 500 psi) pendant une période de temps inférieure à 40 secondes.

**9.** Procédé selon la revendication 1, dans lequel le séchage des morceaux réduit une teneur en humidité des morceaux à environ 6 % à environ 9 % d'humidité.

**10.** Procédé selon la revendication 9, dans lequel le traitement de l'extrudat comprend l'enrobage des morceaux séchés avec au moins l'un parmi de la graisse animale ou un digestat animal.

**11.** Composition alimentaire sèche pour animal de compagnie comprenant 14 % en poids à 20 % en poids de viande qui n'est pas de la farine de viande, dans lequel la viande qui n'est pas de la farine de viande est une bouillie de viande provenant de viande fraîche ou congelée.

**12.** Composition alimentaire sèche pour animal de compagnie selon la revendication 11, ayant une teneur en protéines d'environ 18 % à environ 30 %.

**13.** Composition alimentaire sèche pour animal de compagnie selon la revendication 12, dans laquelle la composition alimentaire sèche pour animal de compagnie comprend la bouillie de viande et une source de protéines choisie dans le groupe constitué d'une protéine végétale, une farine de viande et des combinaisons de celles-ci.

**14.** Composition alimentaire sèche pour animal de compagnie selon la revendication 11, dans laquelle la viande qui n'est pas de la farine de viande est fournie par fabrication de la composition alimentaire sèche pour animal de compagnie avec un processus comprenant la soumission de la viande fraîche ou congelée à une réduction de taille, la cuisson de la viande fraîche ou congelée réduite en taille avec une injection directe de vapeur, l'émulsification de la viande injectée de vapeur pour former une bouillie, et l'ajout de la bouillie à un ou plusieurs autres ingrédients.

**15.** Procédé d'amélioration de la biodisponibilité d'au moins l'un parmi un acide gras essentiel ou un acide aminé essentiel par rapport à une formulation alimentaire sèche initiale pour animal de compagnie comprenant une farine de viande qui fournit au moins une partie d'une teneur en protéines de la formulation alimentaire sèche initiale pour animal de compagnie, le procédé comprenant :

l'ajustement de la formulation alimentaire sèche initiale pour animal de compagnie pour remplacer au moins une partie de la farine de viande par de la viande fournie par une viande qui n'est pas de la farine de viande, dans lequel la viande qui n'est pas de la farine de viande est une bouillie de viande provenant de viande fraîche ou congelée, dans lequel la bouillie de viande représente 14 % en poids à 20 % en poids de la formulation alimentaire ajustée pour animal de compagnie, dans lequel la formulation alimentaire ajustée pour animal de compagnie a une teneur en protéines qui est à peu près égale à la teneur en protéines de la formulation alimentaire sèche initiale pour animal de compagnie ; et
la production d'un granulé selon la formulation alimentaire ajustée pour animal de compagnie.

**16.** Procédé selon la revendication 15, dans lequel la formulation alimentaire sèche initiale pour animal de compagnie comprend des ingrédients non carnés, et la formulation alimentaire ajustée pour animal de compagnie a la même quantité des ingrédients non carnés par rapport à la formulation alimentaire sèche initiale pour animal de compagnie.

**17.** Procédé selon la revendication 15 dans lequel l'acide gras essentiel est l'acide linoléique ou l'acide aminé essentiel est l'arginine ou la lysine.

FIG. 1A

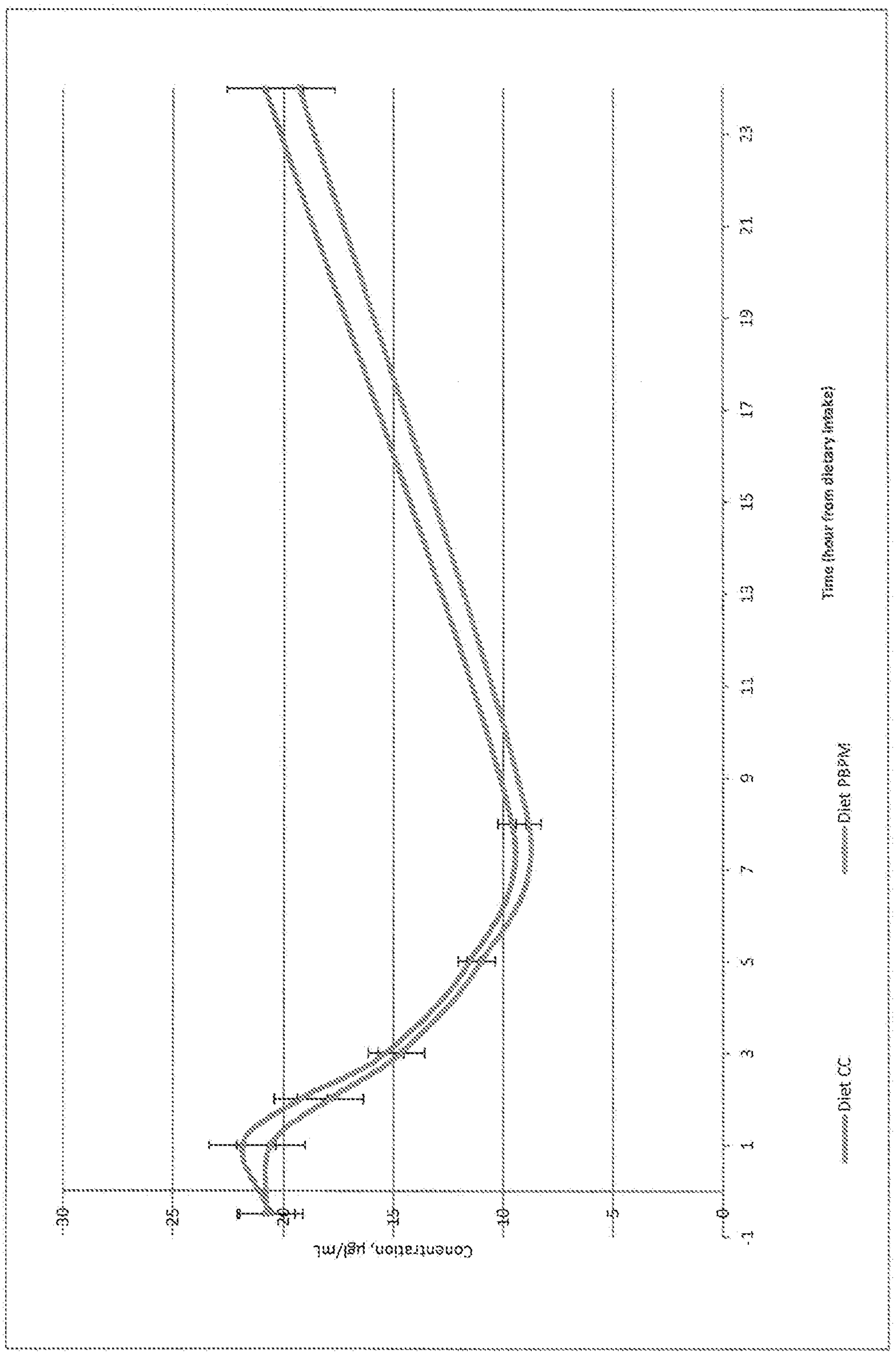
Concentration, µg/mL
30
25
20
15
10
5
0
-1
1
3
5
7
9
11
13
15
17
19
21
23
Time (hour from dietary intake)
Diet CC
Diet PBPM

FIG. 1B

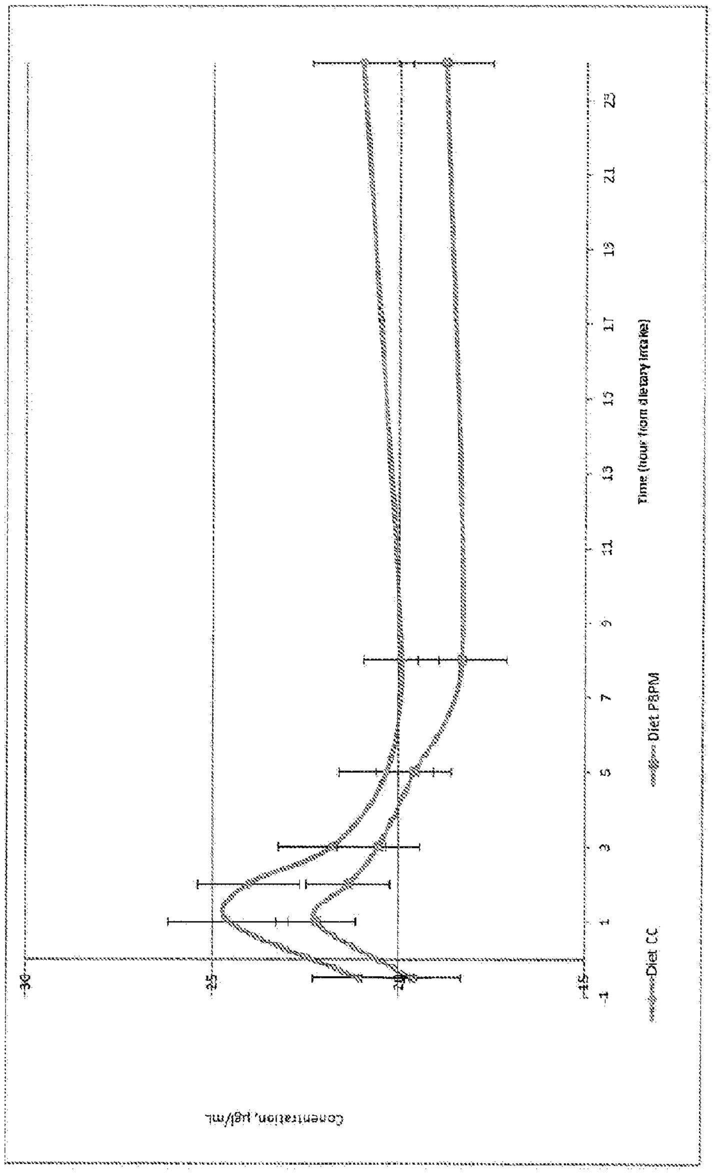
Conentration, µg/mL
30
25
20
15
-1
1
3
5
7
9
11
13
15
17
19
21
23
Time (hour from dietary intake)
Diet CC
Diet PBPM

FIG. 2

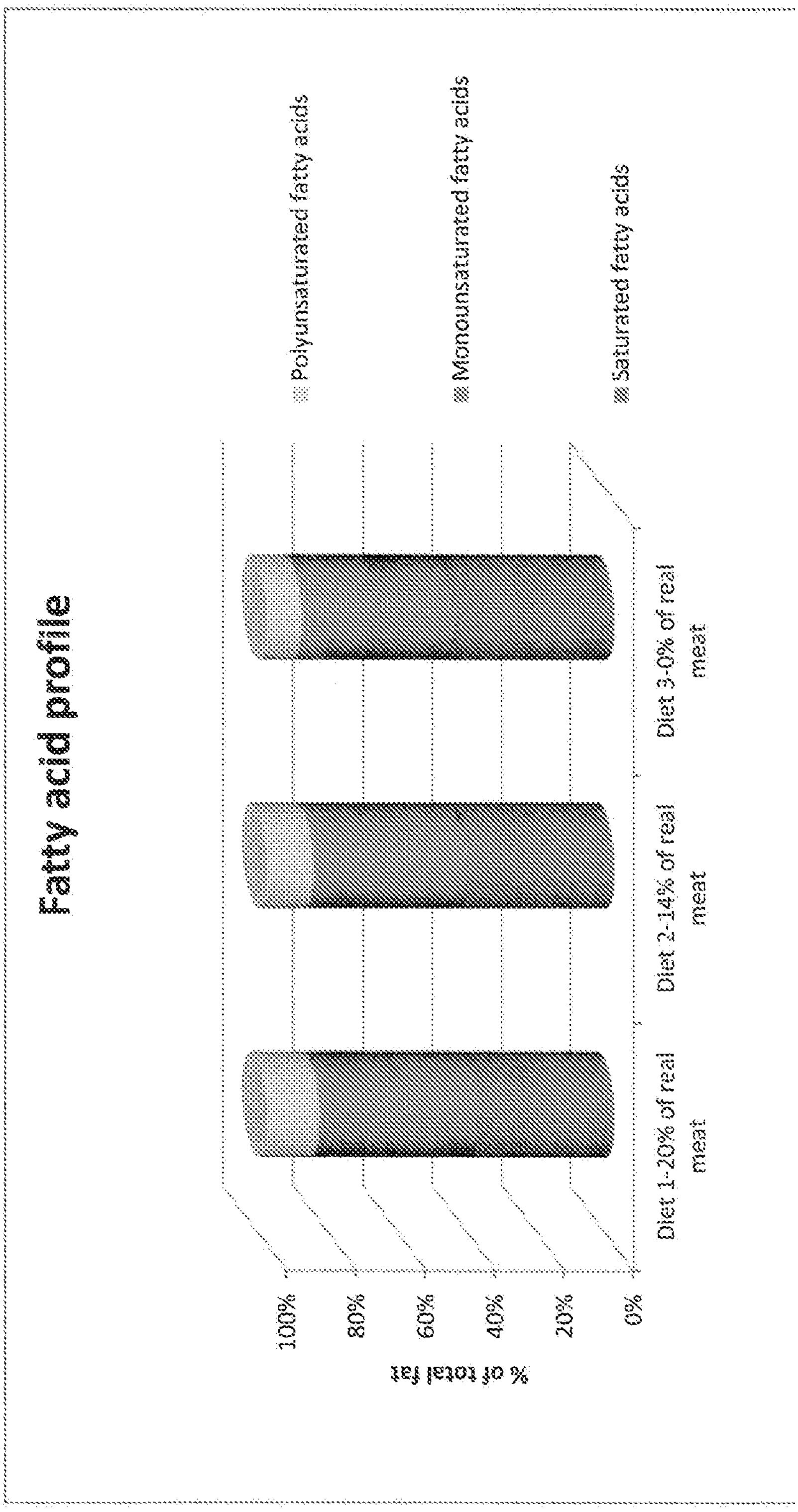
Fatty acid profile
% of total fat
100%
80%
60%
40%
20%
0%
Diet 1-20% of real meat
Diet 2-14% of real meat
Diet 3-0% of real meat
Polyunsaturated fatty acids
Monounsaturated fatty acids
Saturated fatty acids

FIG. 3

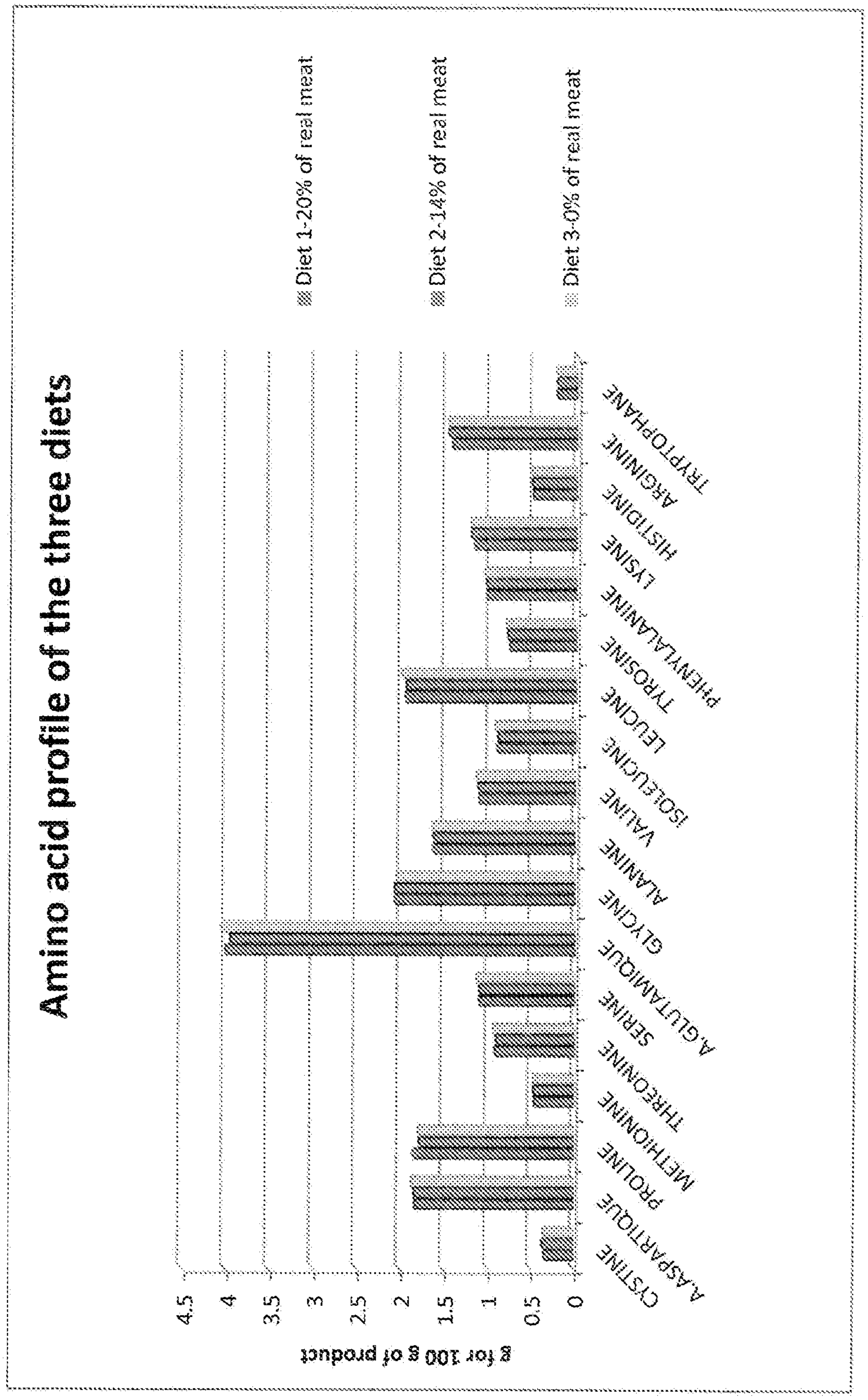
Amino acid profile of the three diets
g for 100 g of product
0
0.5
1
1.5
2
2.5
3
3.5
4
4.5
CYSTINE
A.ASPARTIQUE
PROLINE
METHIONINE
THREONINE
SERINE
A.GLUTAMIQUE
GLYCINE
ALANINE
VALINE
ISOLEUCINE
LEUCINE
TYROSINE
PHENYLALANINE
LYSINE
HISTIDINE
ARGININE
TRYPTOPHANE
Diet 1-20% of real meat
Diet 2-14% of real meat
Diet 3-0% of real meat

FIG. 4

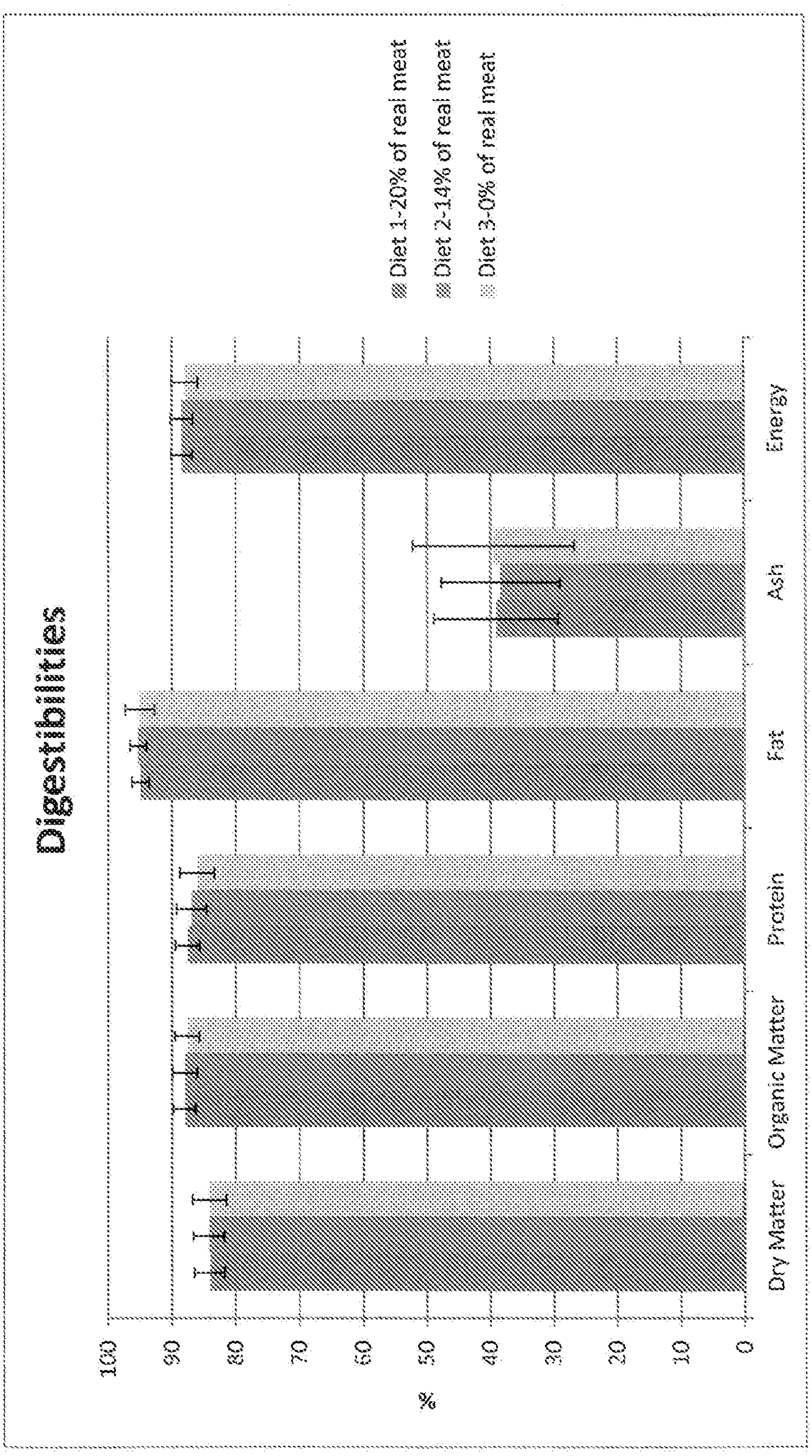
Digestibilities
%
100
90
80
70
60
50
40
30
20
10
0
Dry Matter
Organic Matter
Protein
Fat
Ash
Energy
Diet 1-20% of real meat
Diet 2-14% of real meat
Diet 3-0% of real meat

FIG. 5

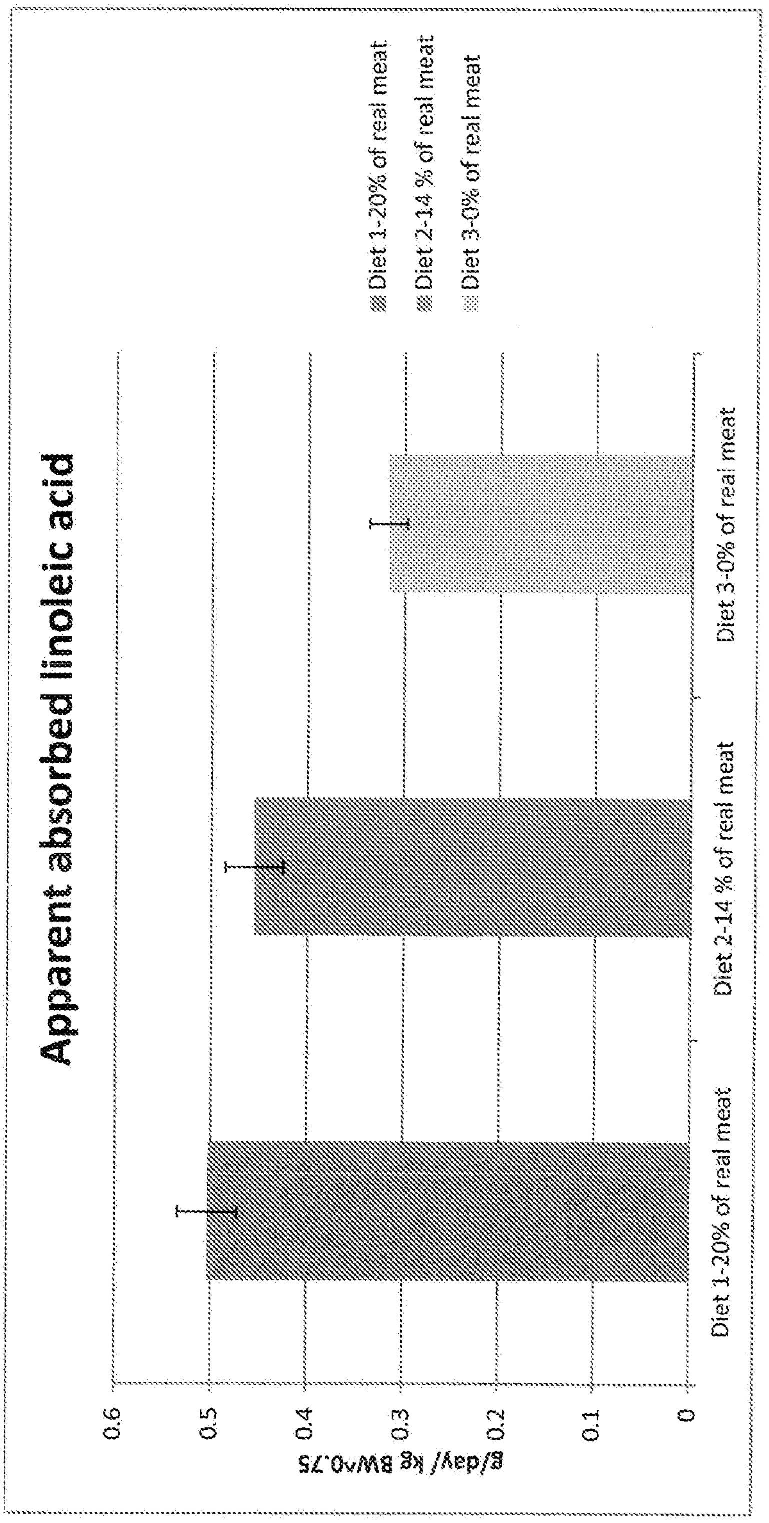
Apparent absorbed linoleic acid
g/day/ kg BW^0.75
0.6
0.5
0.4
0.3
0.2
0.1
0
Diet 1-20% of real meat
Diet 2-14 % of real meat
Diet 3-0% of real meat
Diet 1-20% of real meat
Diet 2-14 % of real meat
Diet 3-0% of real meat

FIG. 6

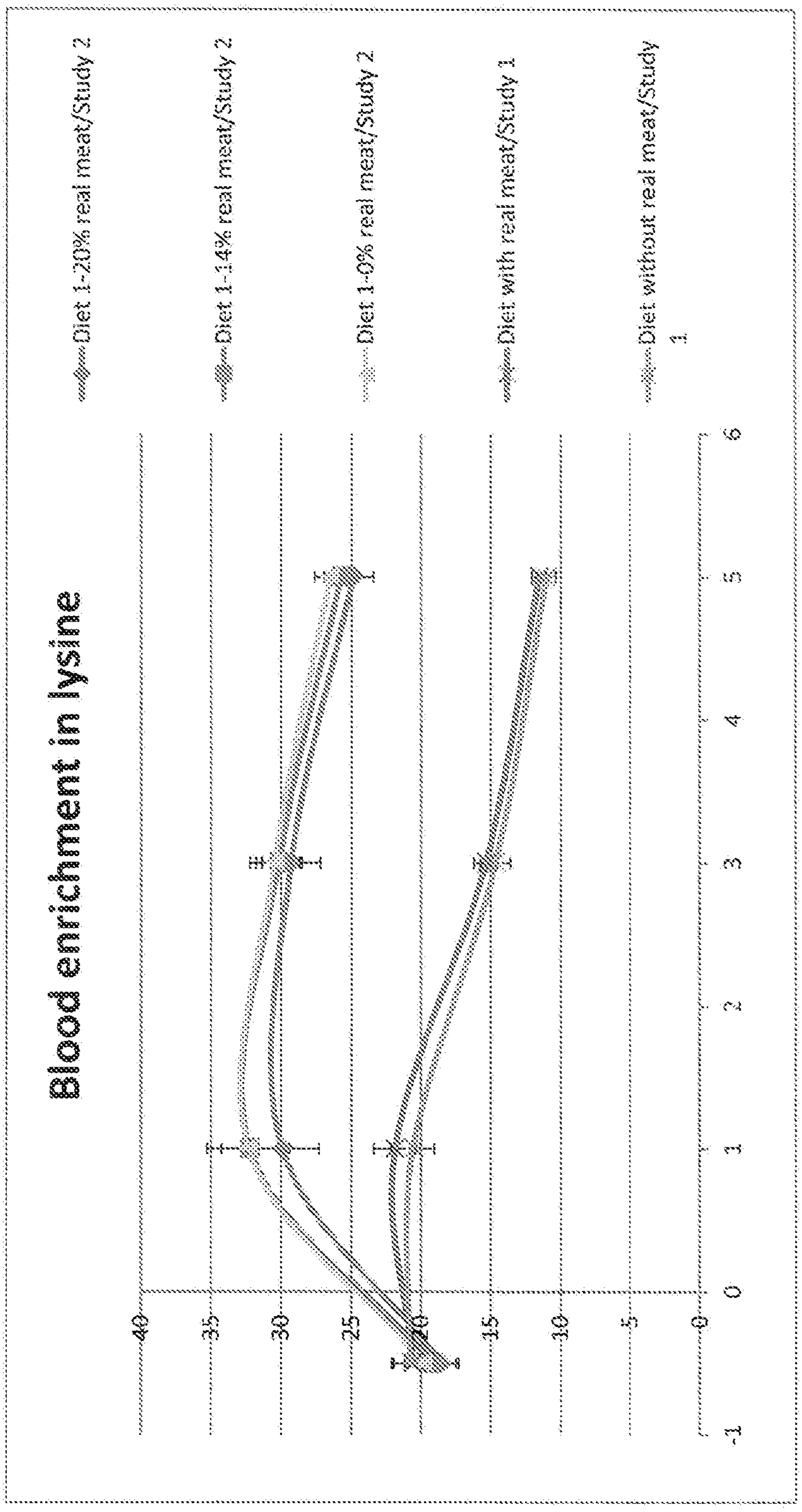
Blood enrichment in lysine
40
35
30
25
20
15
10
5
0
-1
0
1
2
3
4
5
6
Diet 1-20% real meat/Study 2
Diet 1-14% real meat/Study 2
Diet 1-0% real meat/Study 2
Diet with real meat/Study 1
Diet without real meat/Study 1

FIG. 7

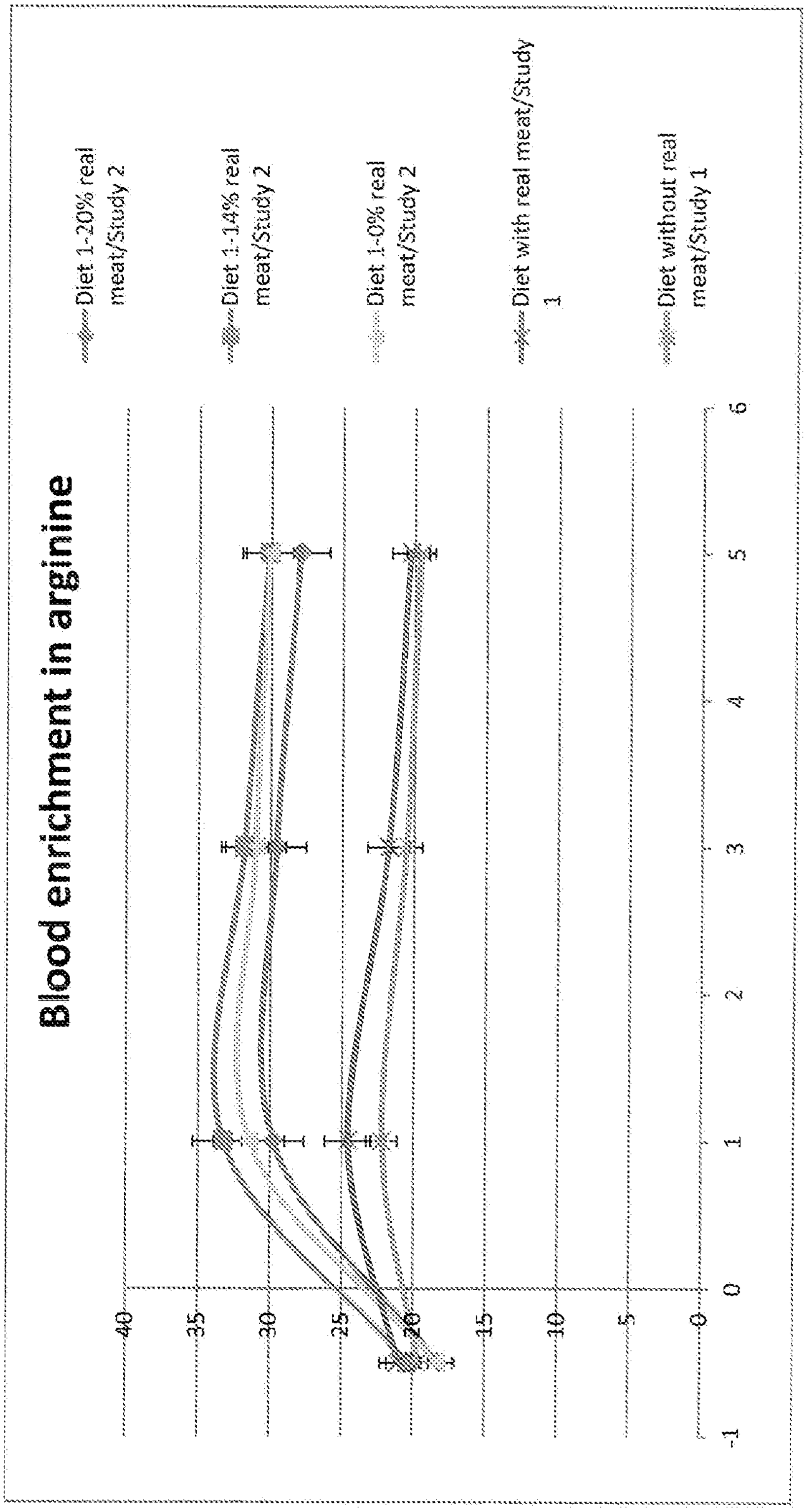
Blood enrichment in arginine
40
35
30
25
20
15
10
5
0
-1
0
1
2
3
4
5
6
Diet 1-20% real meat/Study 2
Diet 1-14% real meat/Study 2
Diet 1-0% real meat/Study 2
Diet with real meat/Study 1
Diet without real meat/Study 1

FIG. 8

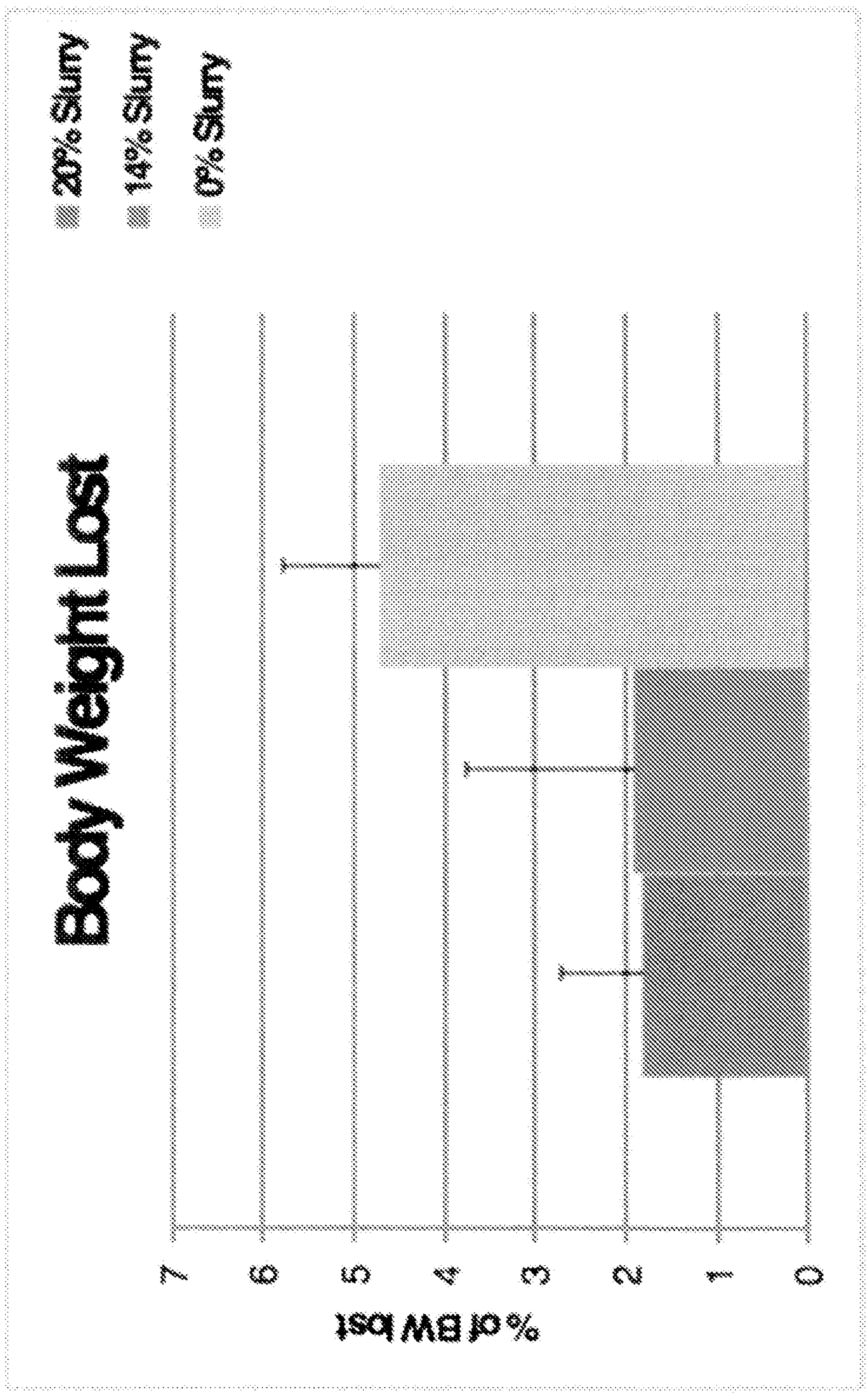
Body Weight Lost
20% Slurry
14% Slurry
0% Slurry
% of BW lost
0
1
2
3
4
5
6
7

FIG. 9

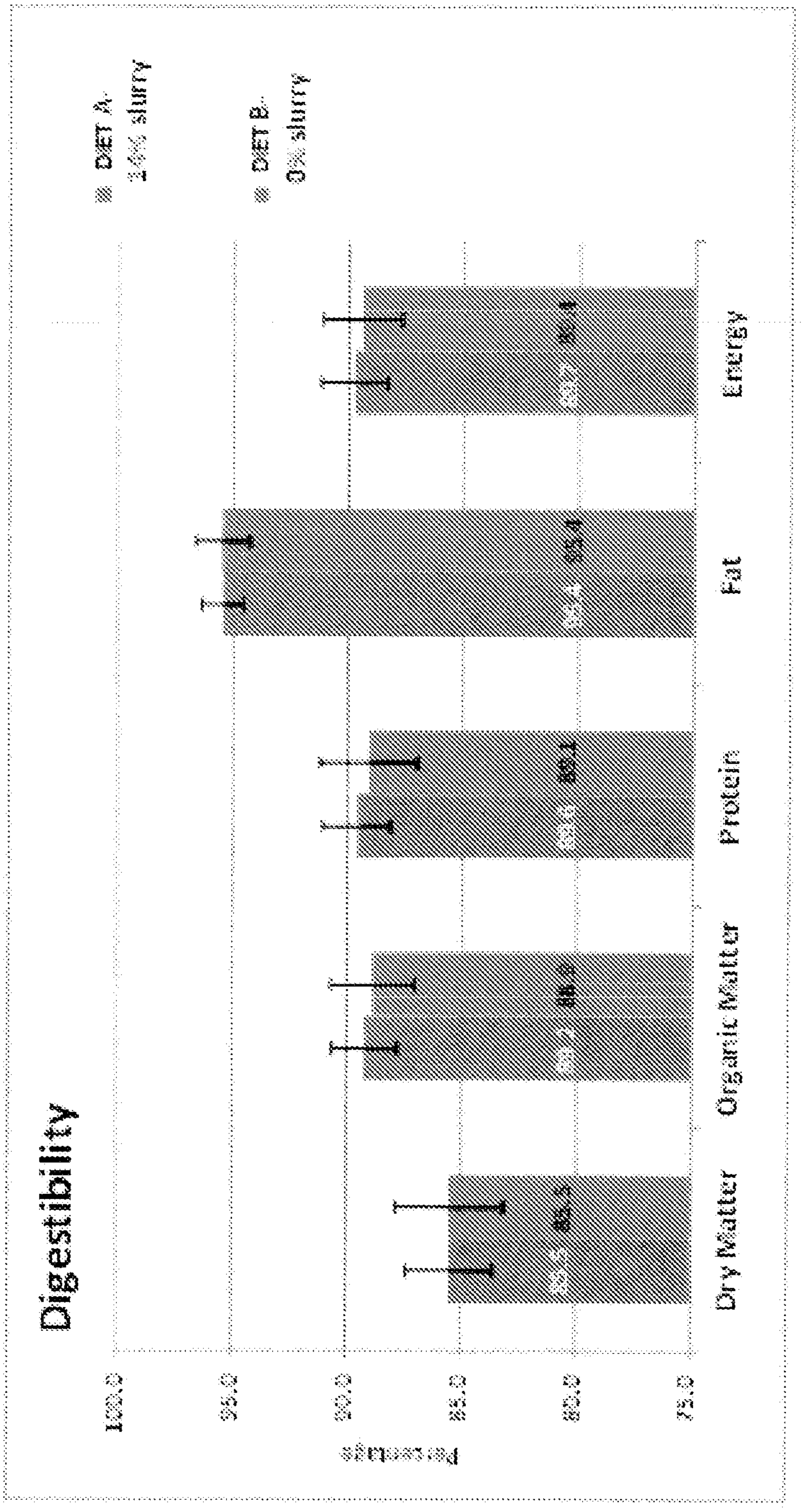
Digestibility
Percentage
100.0
95.0
90.0
85.0
80.0
75.0
Dry Matter
Organic Matter
Protein
Fat
Energy
DIET A-
DIET B-
0% slurry

FIG. 10A

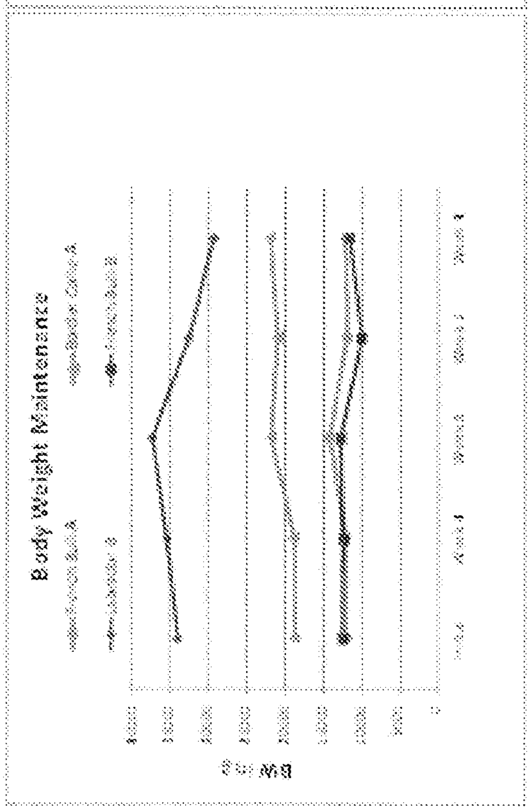


FIG. 10B

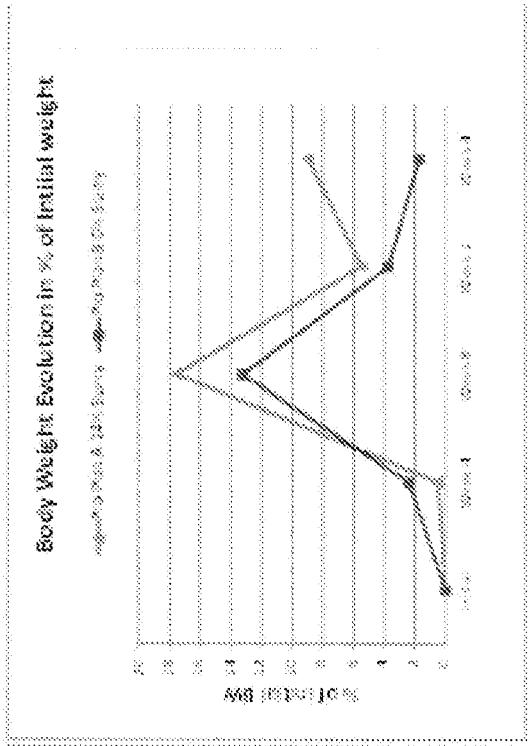

FIG. 11

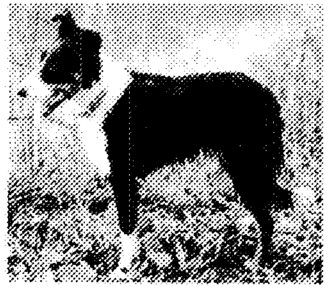

Etna B. Collie Initial

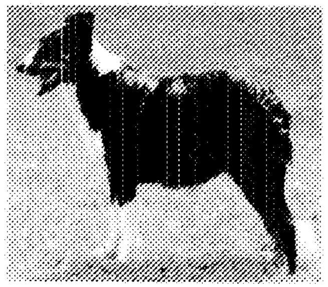

Etna B. Collie 8 weeks

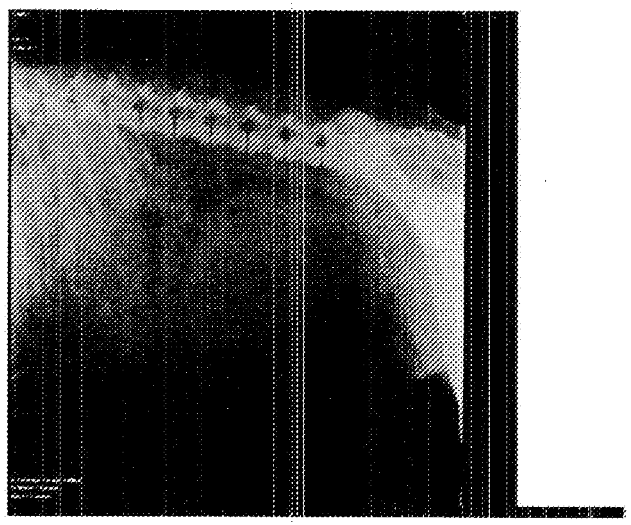

FIG. 12A

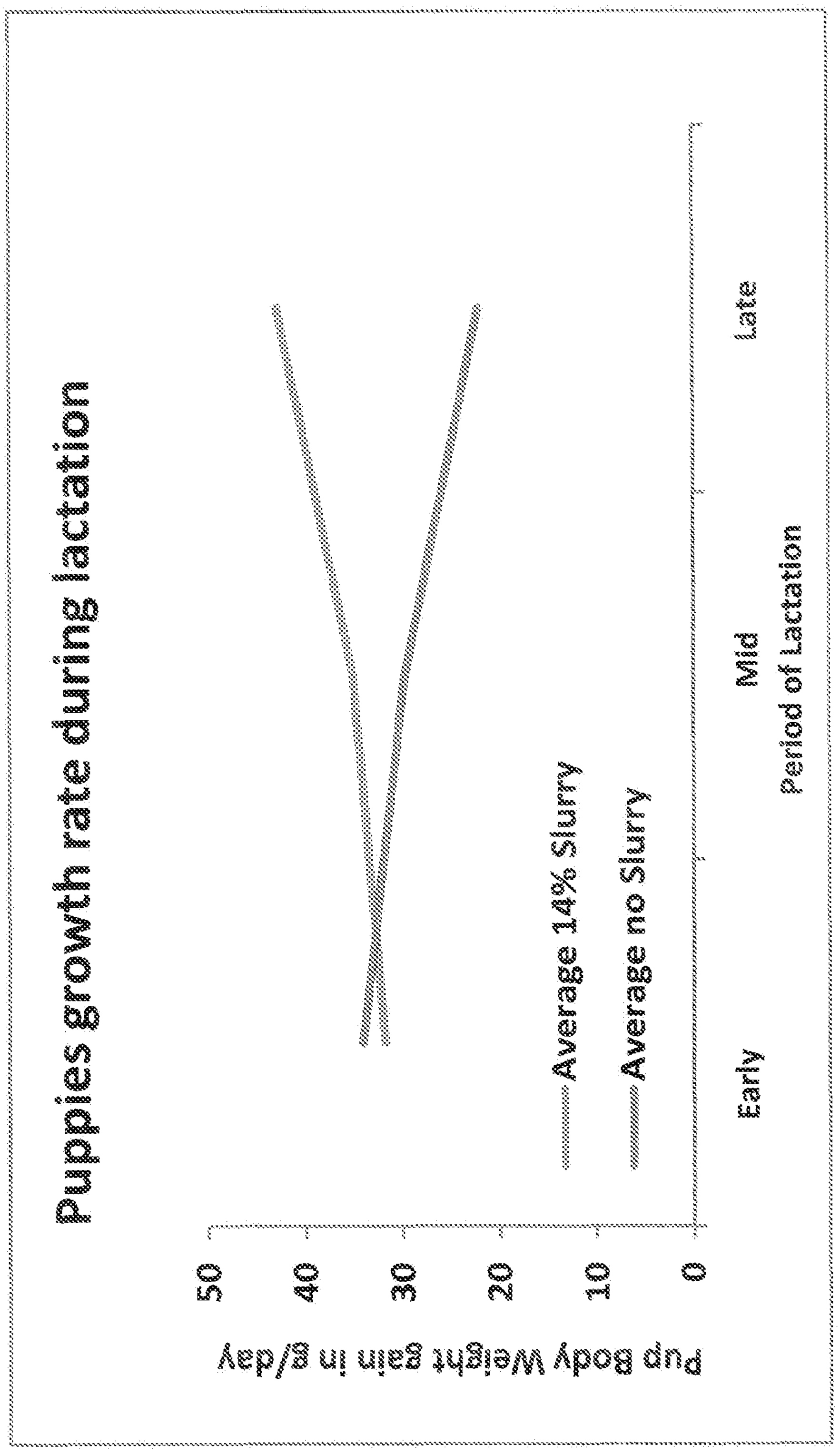

Puppies growth rate during lactation
Pup Body Weight gain in g/day
50
40
30
20
10
0
Early
Mid
Late
Period of Lactation
Average 14% Slurry
Average no Slurry

FIG. 12B

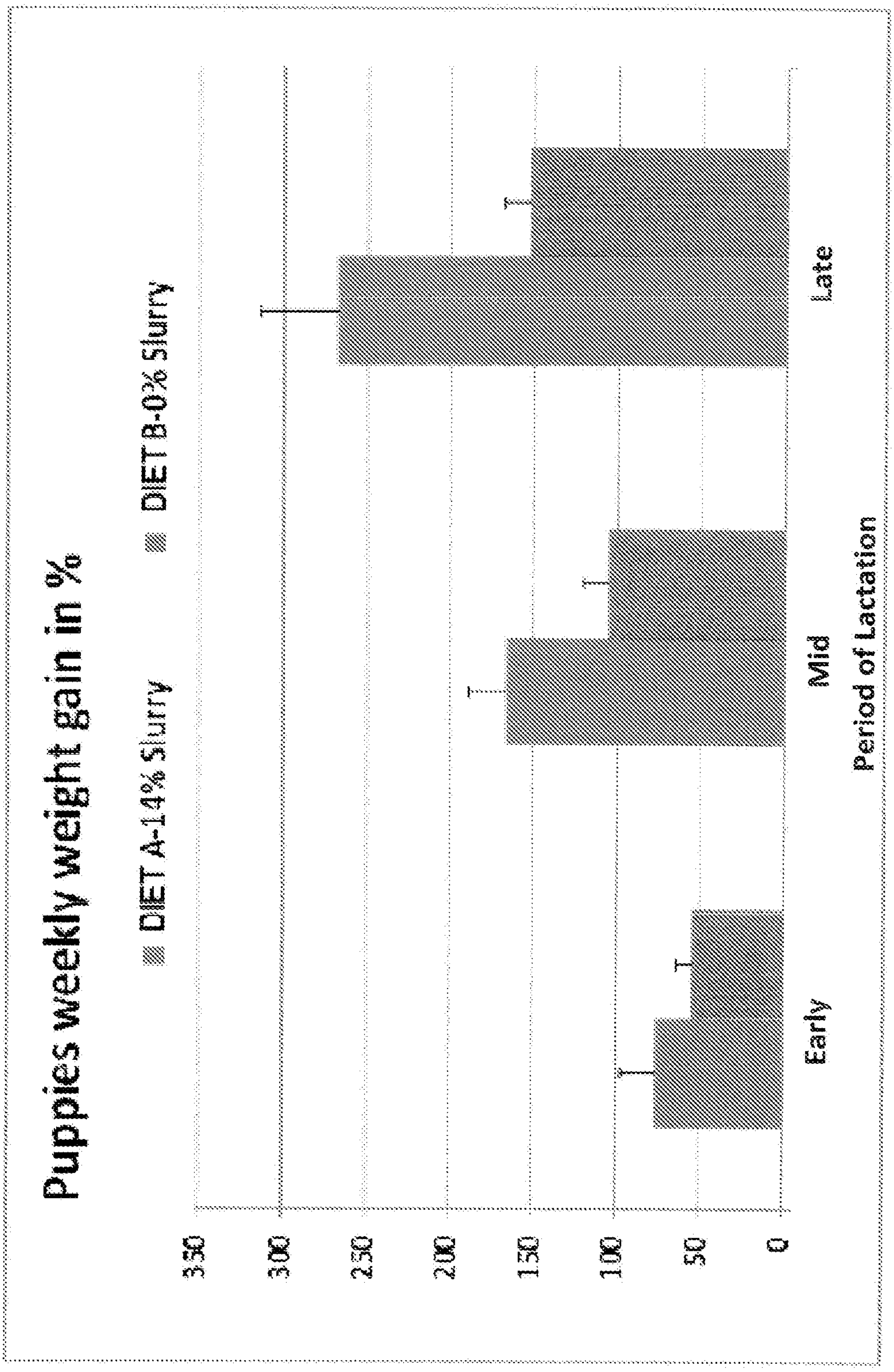
Puppies weekly weight gain in %
DIET A-14% Slurry
DIET B-0% Slurry
350
300
250
200
150
100
50
0
Early
Mid
Late
Period of Lactation

FIG. 13

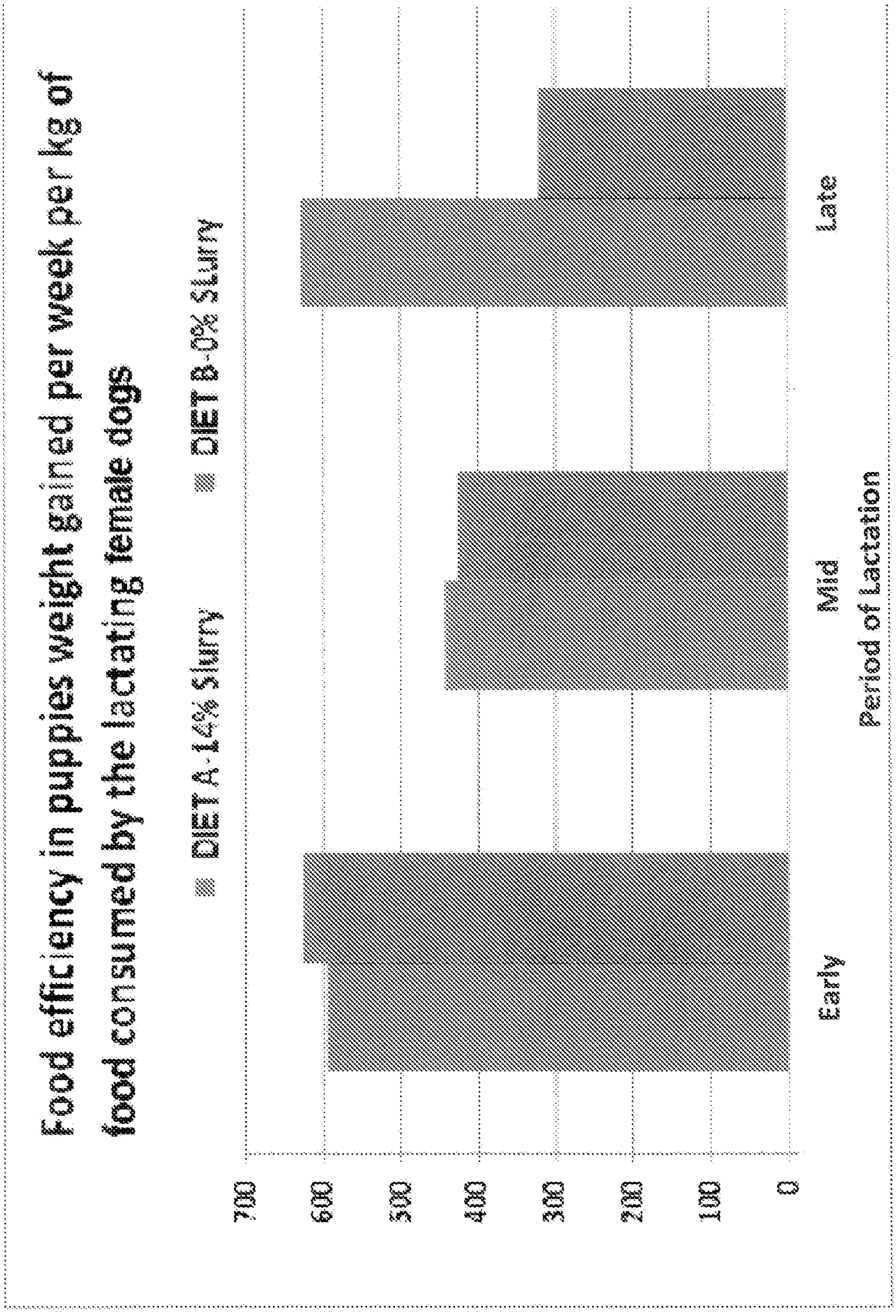
Food efficiency in puppies weight gained per week per kg of food consumed by the lactating female dogs
DIET A-14% Slurry
DIET B-0% Slurry
700
600
500
400
300
200
100
0
Early
Mid
Late
Period of Lactation

FIG. 14A

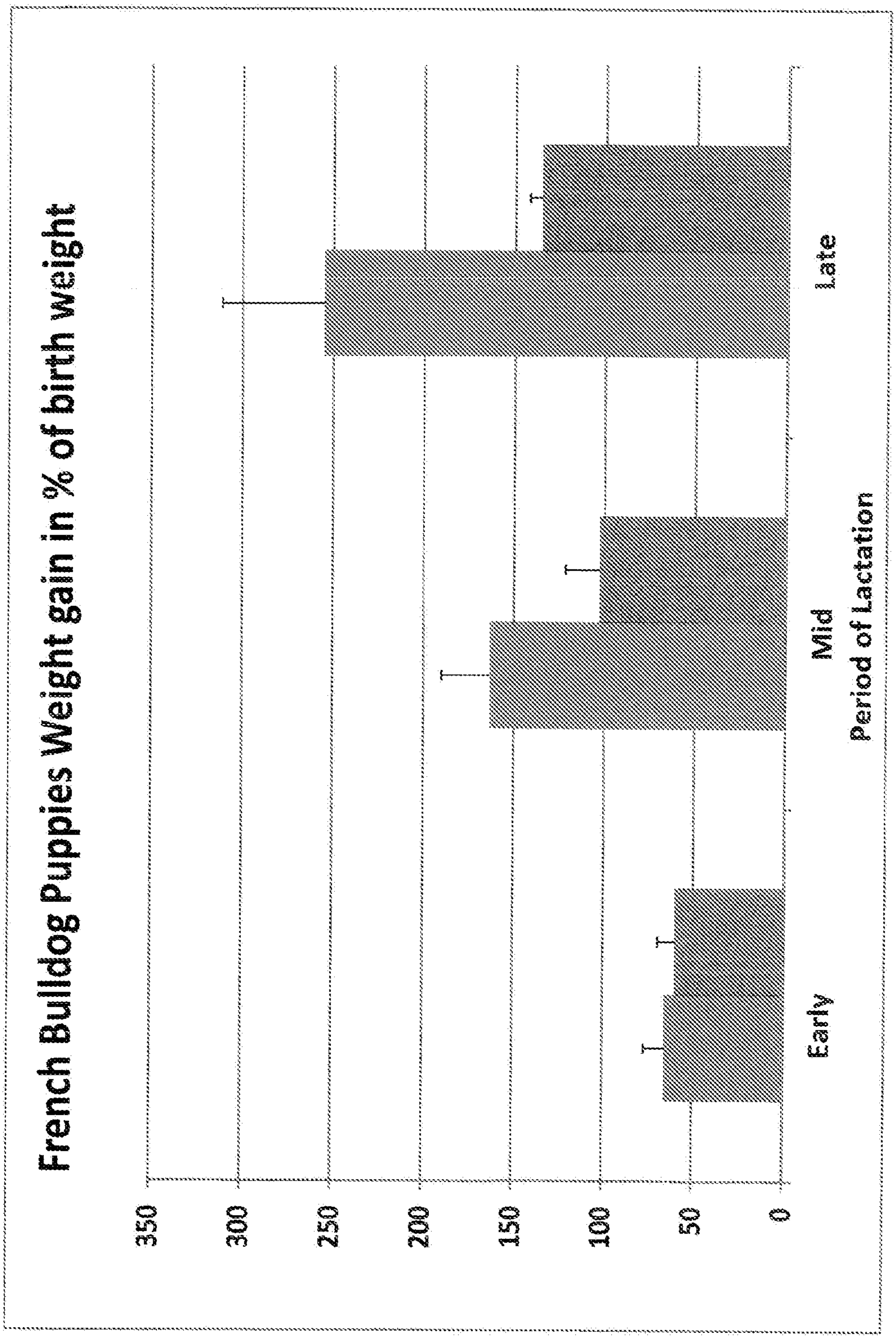

French Bulldog Puppies Weight gain in % of birth weight
350
300
250
200
150
100
50
0
Early
Mid
Late
Period of Lactation

FIG. 14B

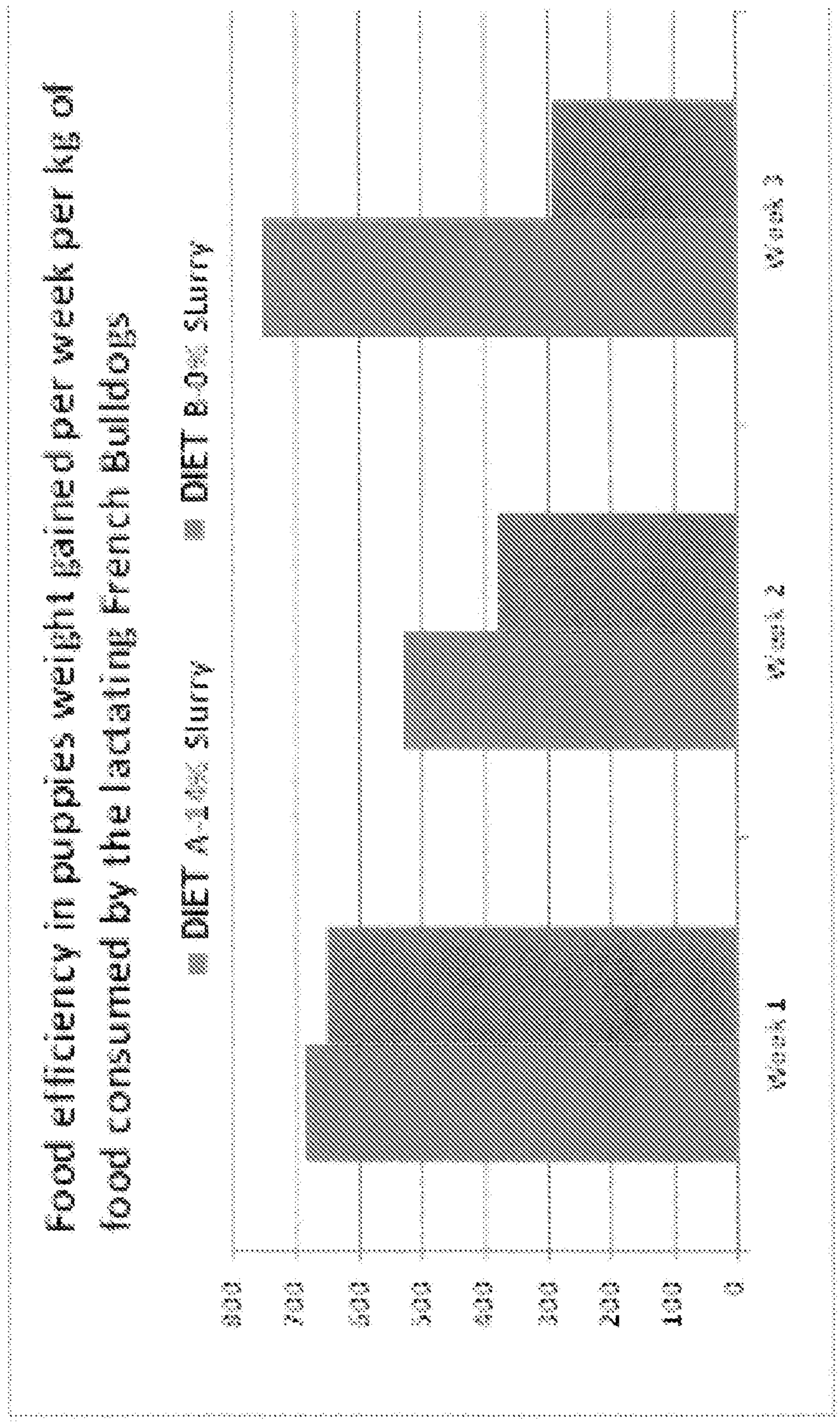
Food efficiency in puppies weight gained per week per kg of food consumed by the lactating French Bulldogs
DIET A-1% Slurry
DIET B-0% Slurry
800
700
600
500
400
300
200
100
0
Week 1
Week 2
Week 3

FIG. 15A

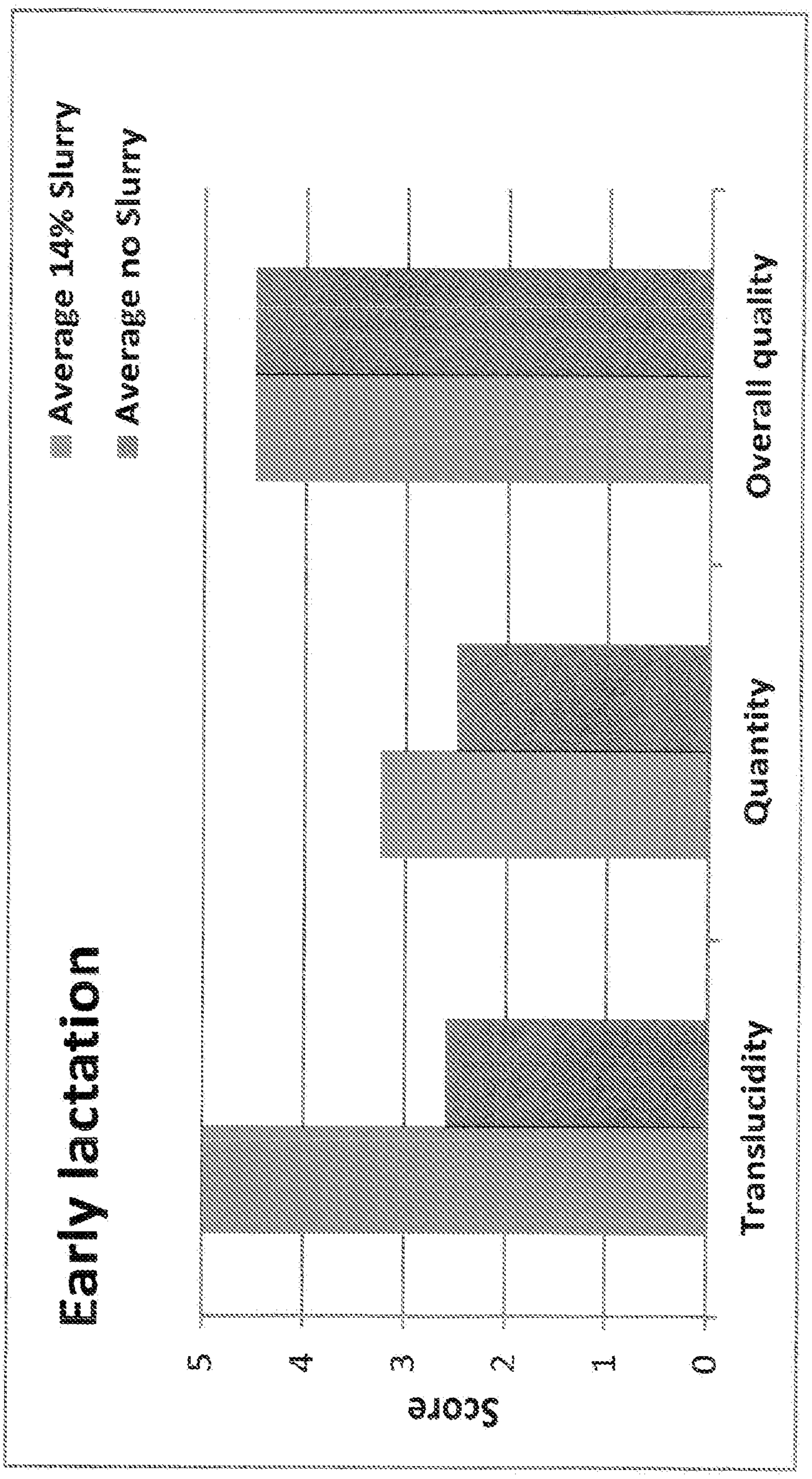
Early lactation
Average 14% Slurry
Average no Slurry
Score
5
4
3
2
1
0
Translucidity
Quantity
Overall quality

FIG. 15B

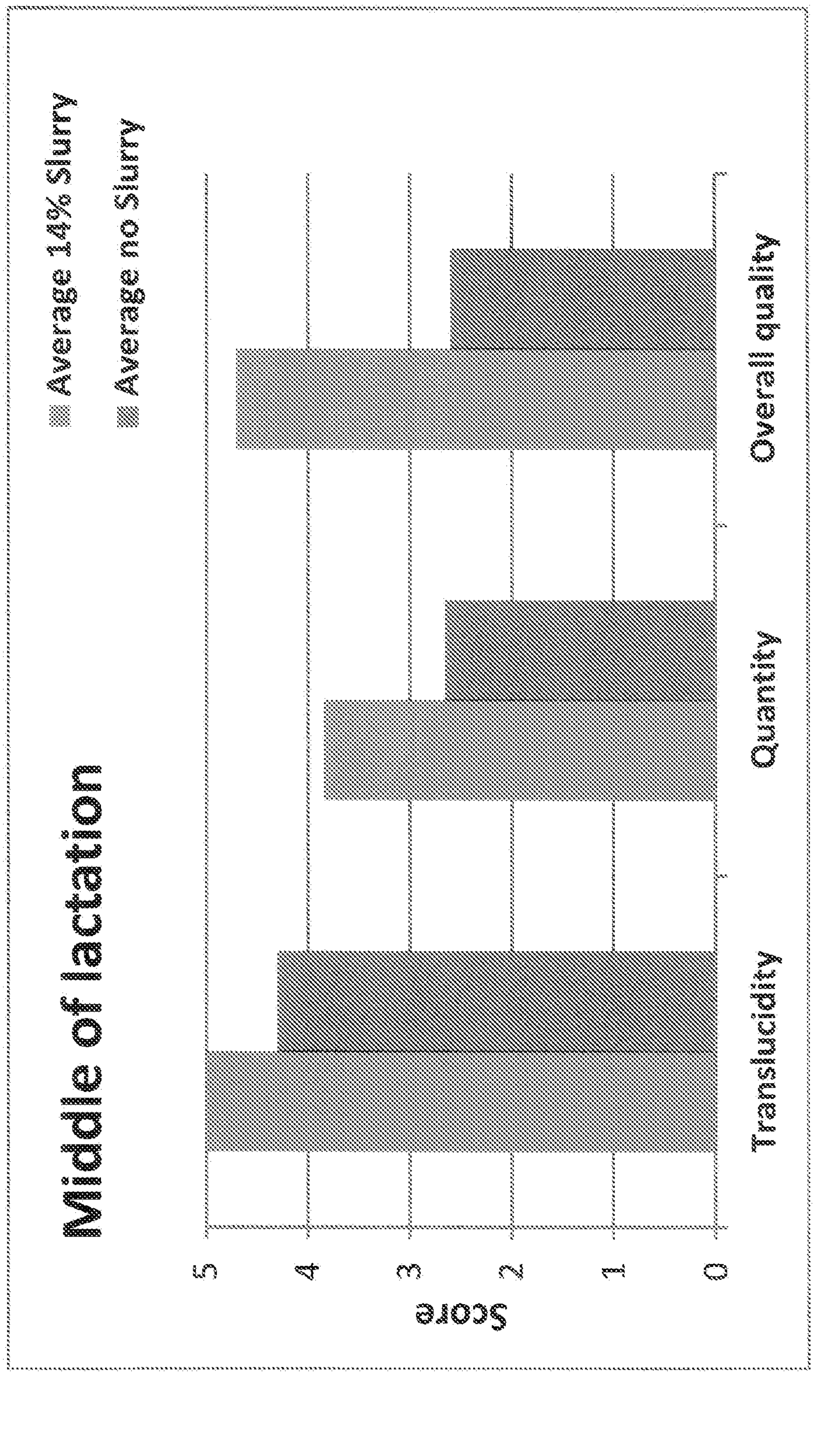
Middle of lactation
Average 14% Slurry
Average no Slurry
Score
0
1
2
3
4
5
Translucidity
Quantity
Overall quality

FIG. 15C

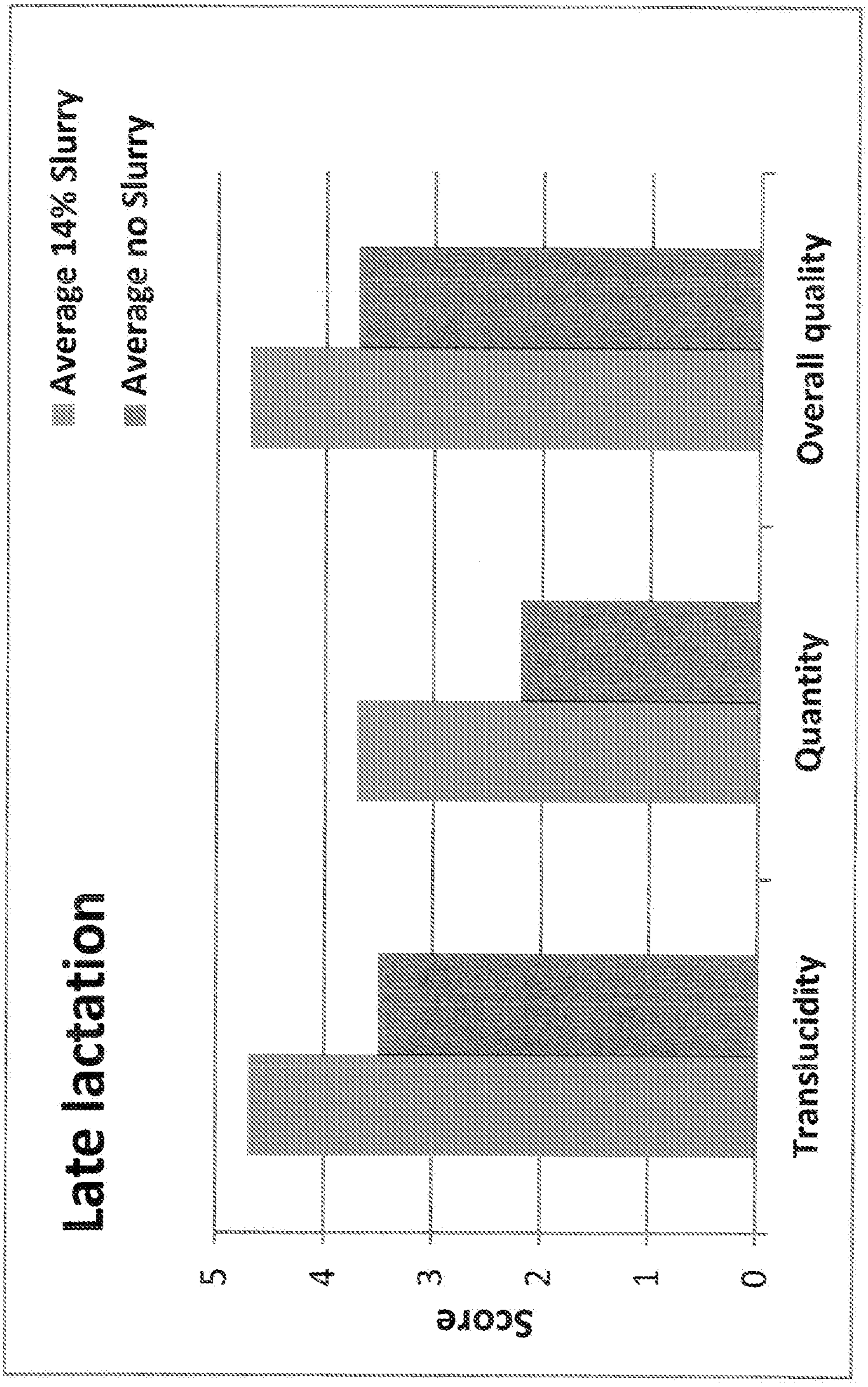
Late lactation
Score
0
1
2
3
4
5
Translucidity
Quantity
Overall quality
Average 14% Slurry
Average no Slurry

FIG. 16

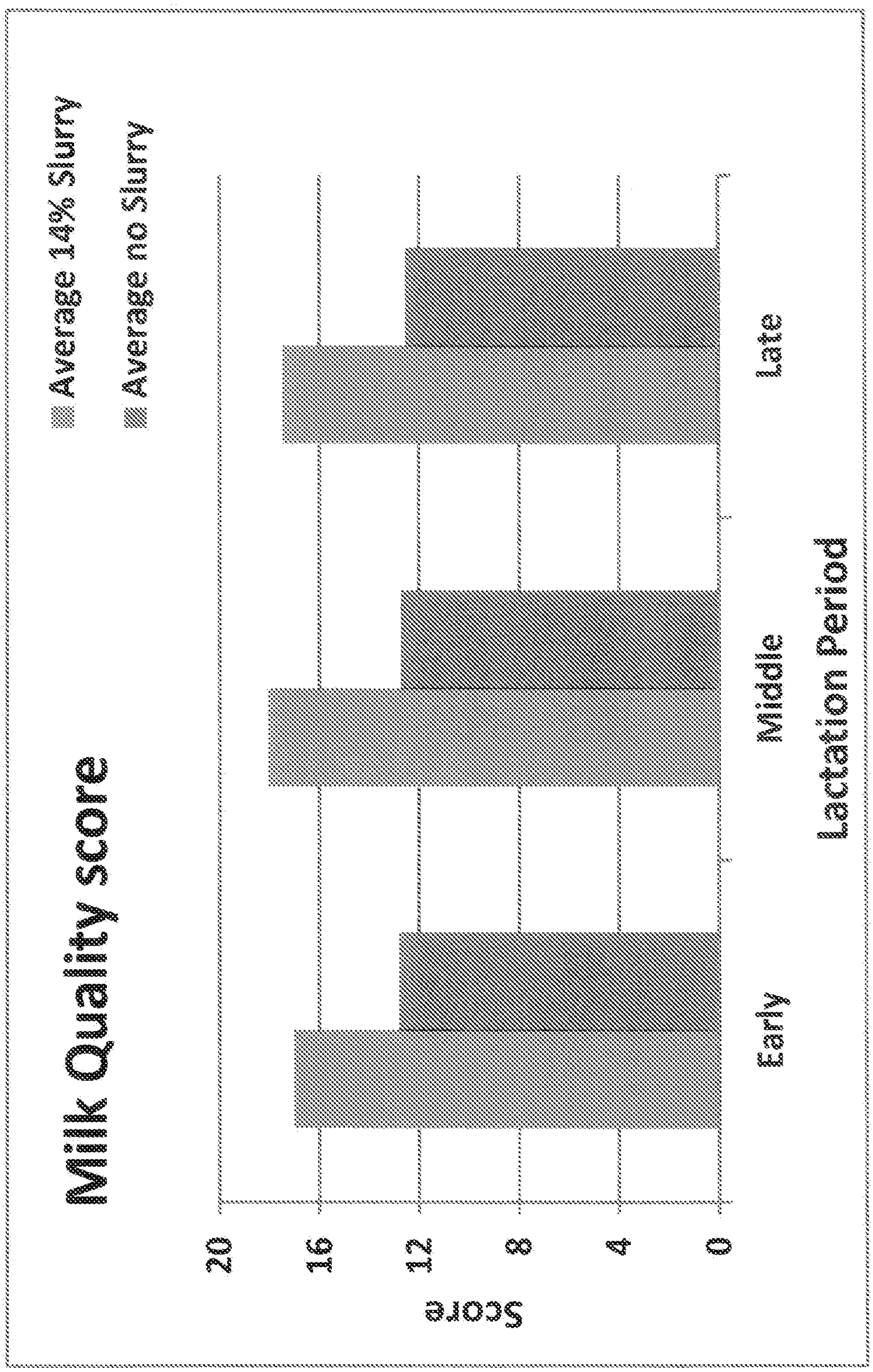
Milk Quality score
Average 14% Slurry
Average no Slurry
Score
20
16
12
8
4
0
Early
Middle
Late
Lactation Period

FIG. 17

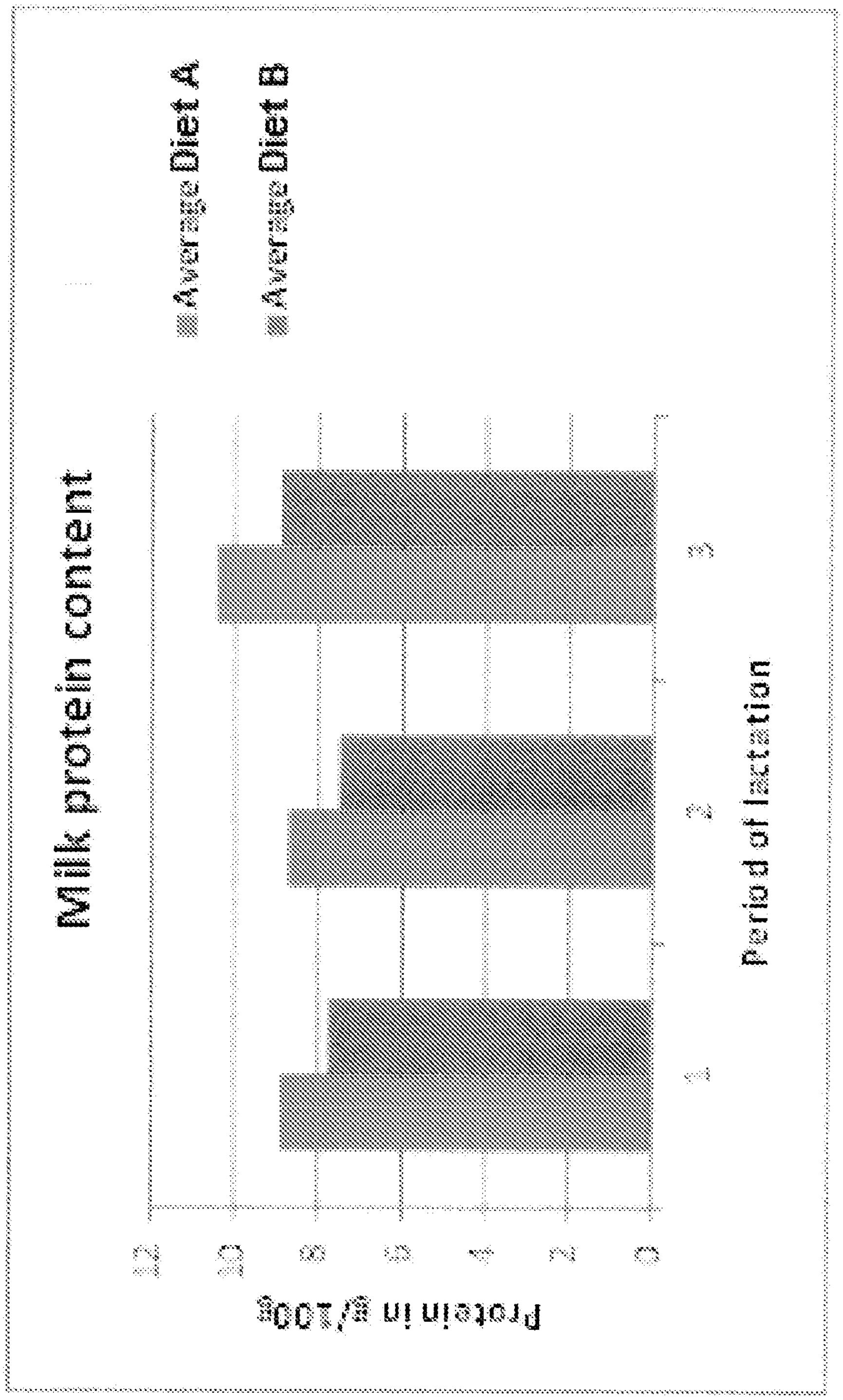
Milk protein content
Average Diet A
Average Diet B
Protein in g/100g
12
10
8
6
4
2
0
1
2
3
Period of lactation

FIG. 18

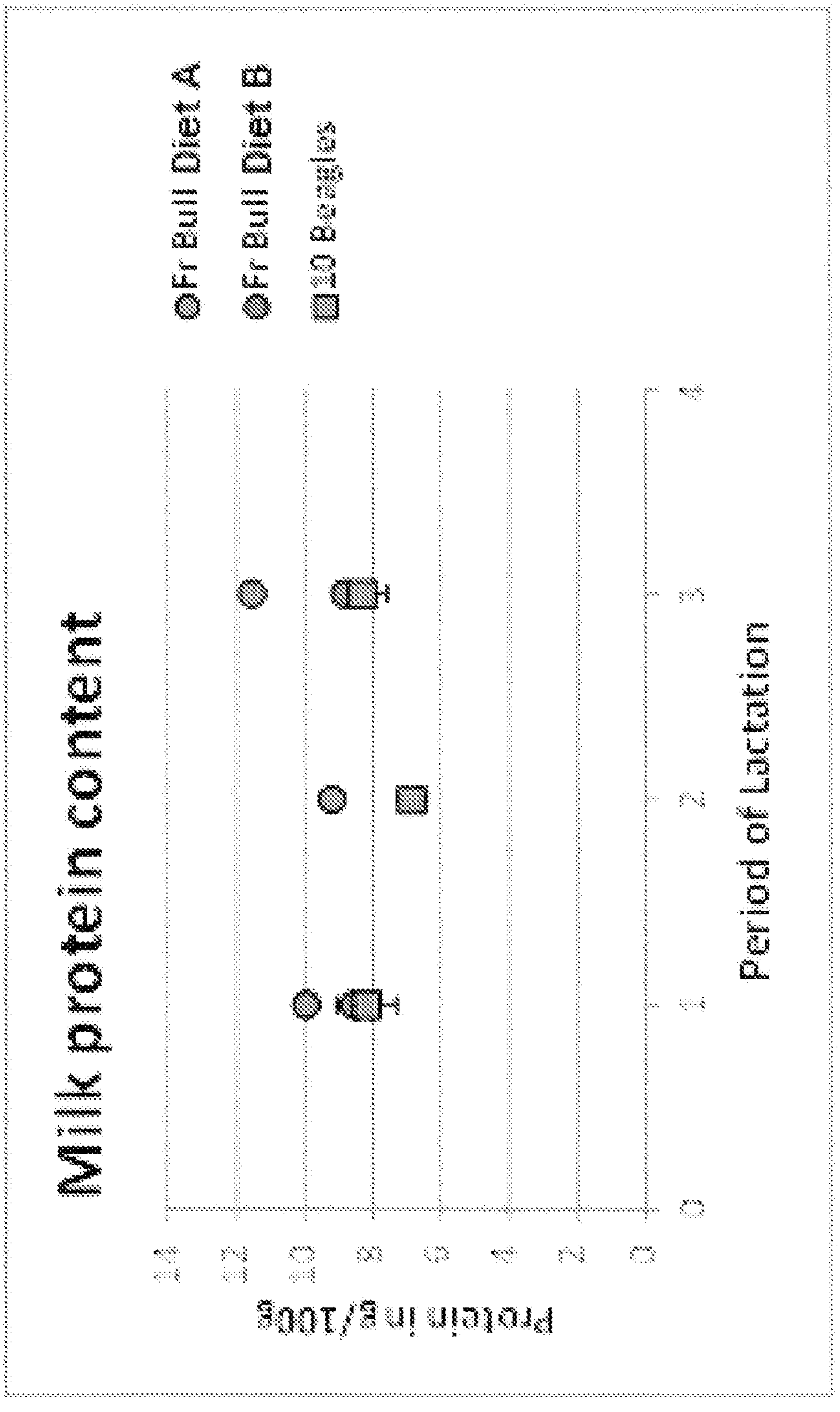
Milk protein content
Protein in g/100g
14
12
10
8
6
4
2
0
0
1
2
3
4
Period of Lactation
Fr Bull Diet A
Fr Bull Diet B
10 Beagles

FIG. 19

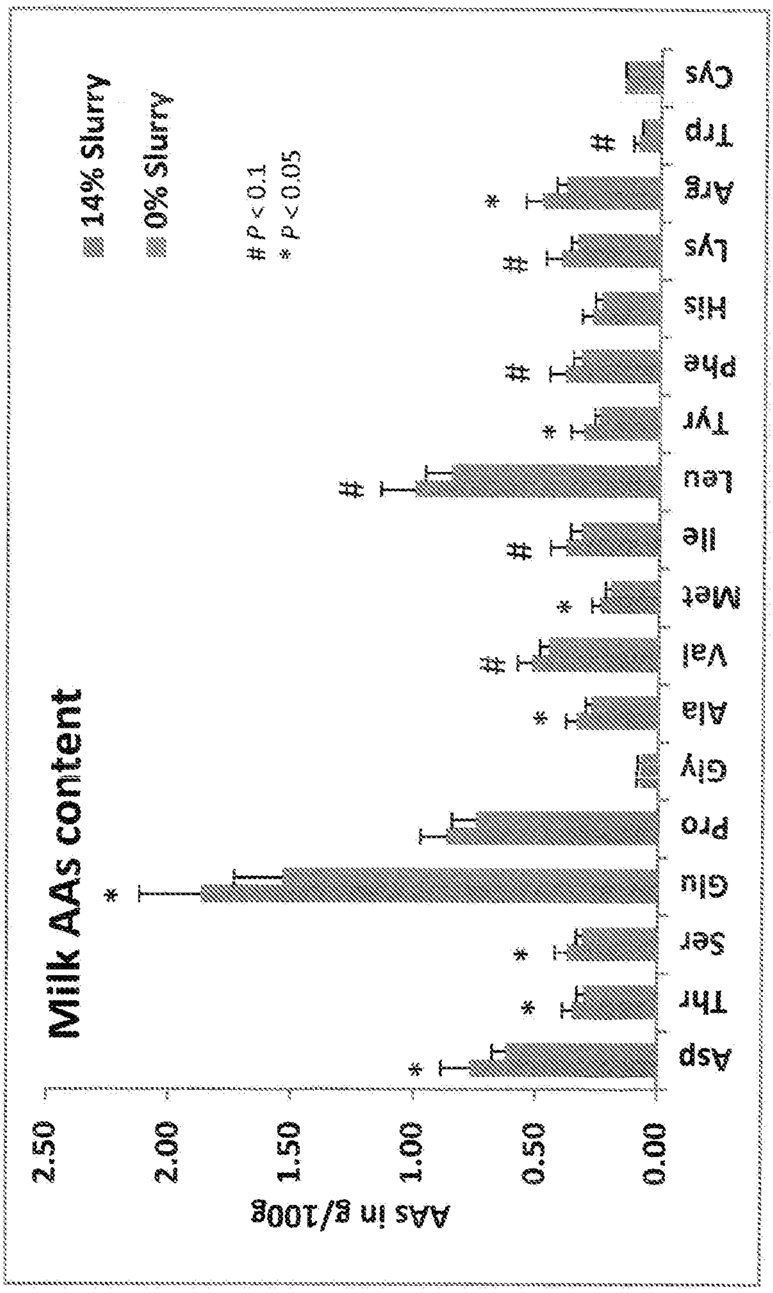
Milk AAs content
14% Slurry
0% Slurry
# P < 0.1
* P < 0.05
AAs in g/100g
2.50
2.00
1.50
1.00
0.50
0.00
Asp
Thr
Ser
Glu
Pro
Gly
Ala
Val
Met
Ile
Leu
Tyr
Phe
His
Lys
Arg
Trp
Cys

FIG. 20A

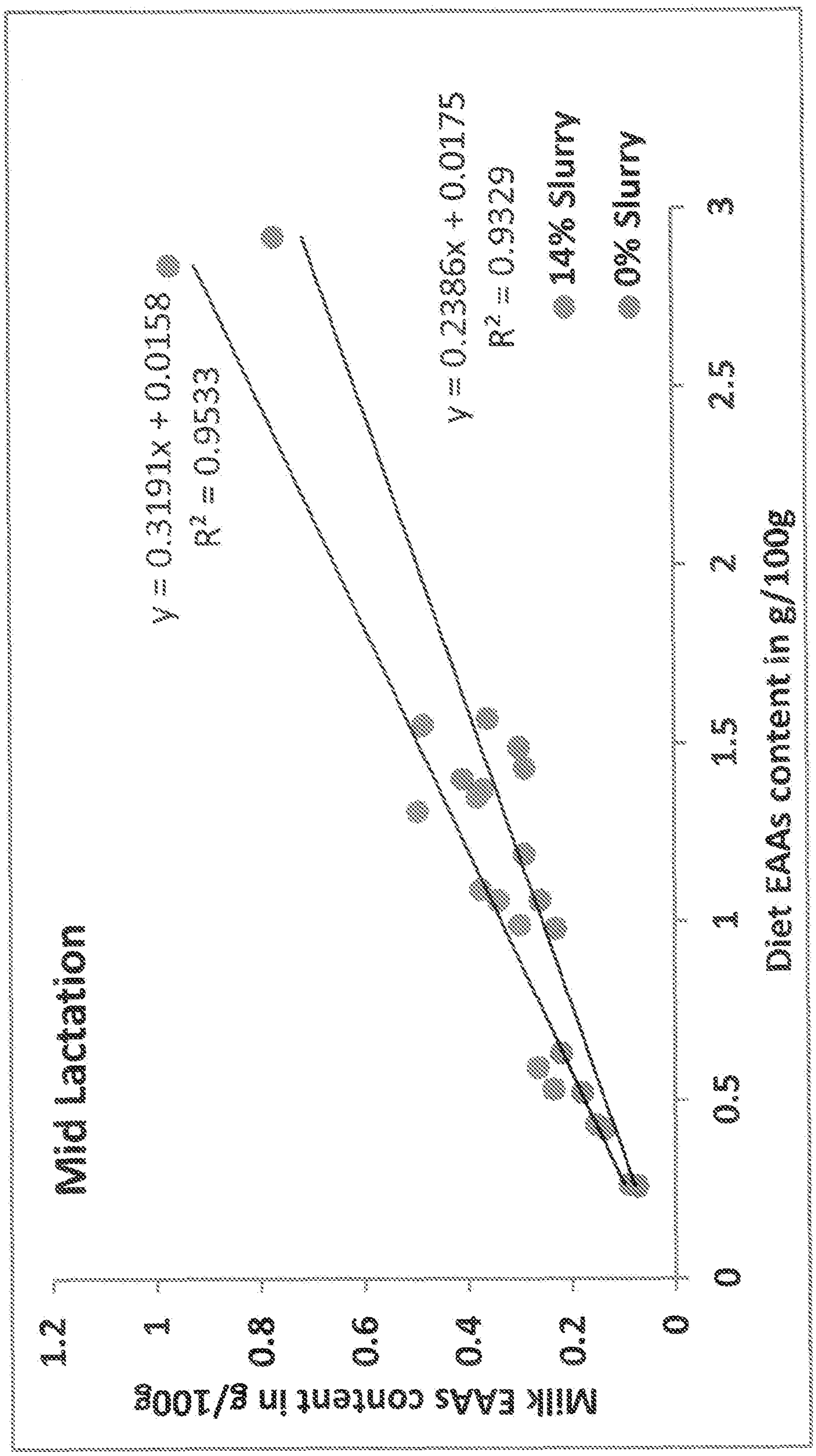
Mid Lactation
Milk EAAs content in g/100g
1.2
1
0.8
0.6
0.4
0.2
0
0
0.5
1
1.5
2
2.5
3
Diet EAAs content in g/100g
y = 0.3191x + 0.0158
$R^2$ = 0.9533
y = 0.2386x + 0.0175
$R^2$ = 0.9329
14% Slurry
0% Slurry

FIG. 20B

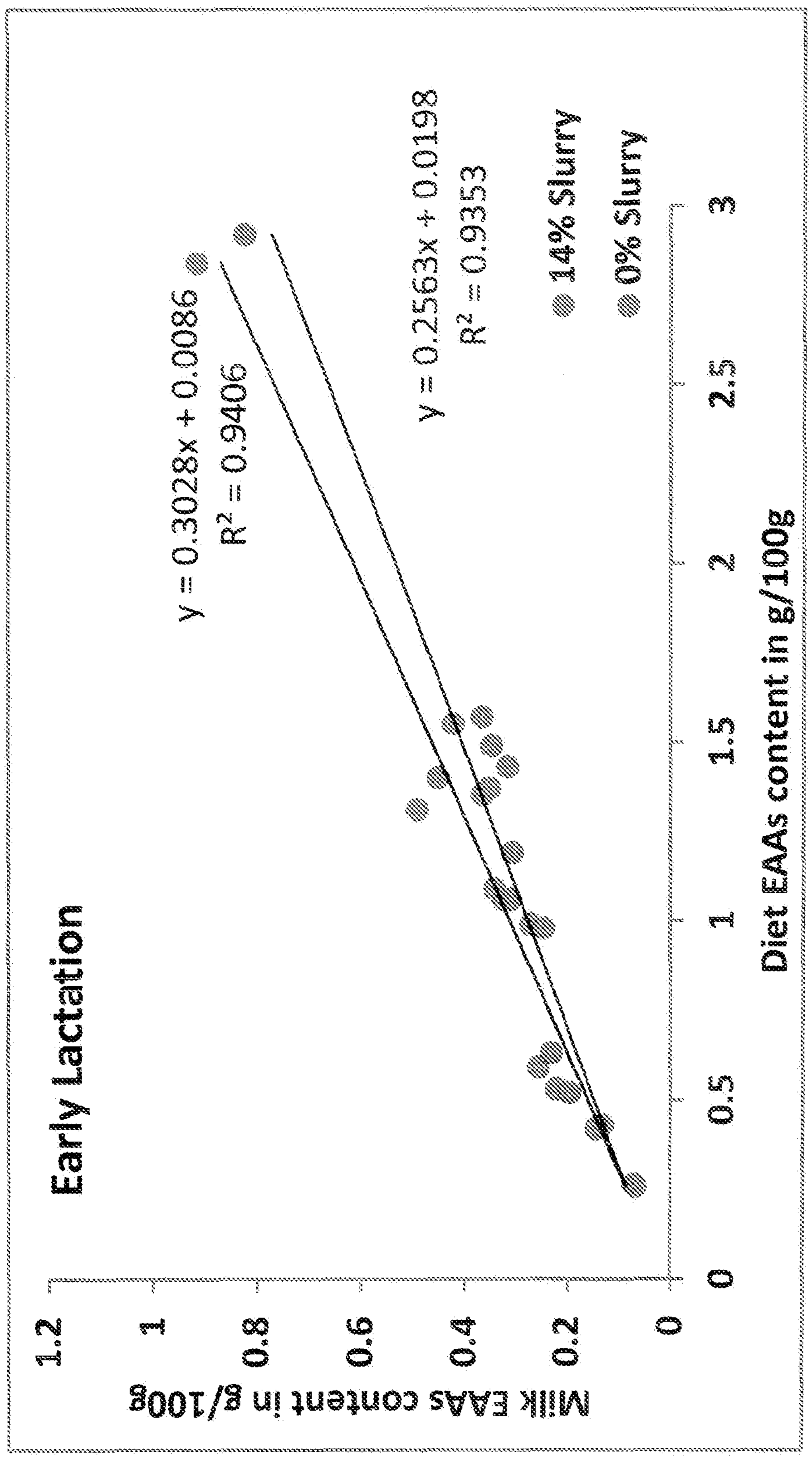
Early Lactation
y = 0.3028x + 0.0086
R² = 0.9406
y = 0.2563x + 0.0198
R² = 0.9353
14% Slurry
0% Slurry
Milk EAAs content in g/100g
1.2
1
0.8
0.6
0.4
0.2
0
Diet EAAs content in g/100g
0
0.5
1
1.5
2
2.5
3

FIG. 20C

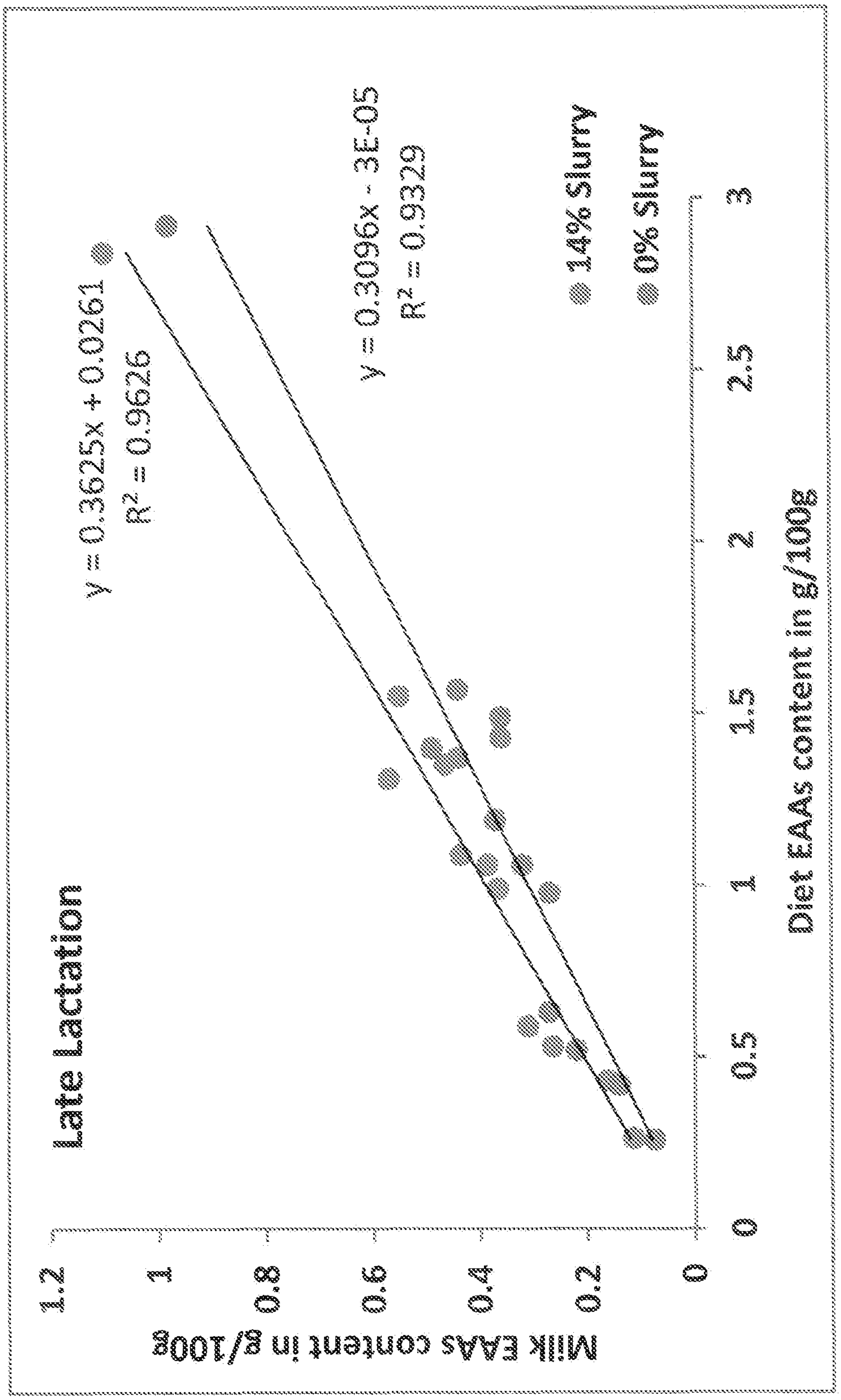
Late Lactation
y = 0.3625x + 0.0261
R² = 0.9626
y = 0.3096x - 3E-05
R² = 0.9329
14% Slurry
0% Slurry
Milk EAAs content in g/100g
Diet EAAs content in g/100g
0
0.2
0.4
0.6
0.8
1
1.2
0
0.5
1
1.5
2
2.5
3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120237642 A1